(19) Europäisches Patentamt
European Patent Office
Office européen des brevets

(11) EP 0 946 504 B1

(12) **FASCICULE DE BREVET EUROPEEN**

(45) Date de publication et mention de la délivrance du brevet: **13.08.2003 Bulletin 2003/33**

(21) Numéro de dépôt: **97947074.7**

(22) Date de dépôt: **17.11.1997**

(51) Int Cl.7: **C07C 319/14**, C07C 323/04, C07C 323/20, C07C 391/02, C07C 29/38, C07C 29/62, C07C 33/46, C07C 33/48, C07C 35/52, C07F 7/18, C07C 41/30, C07C 43/313, C07C 45/42, C07C 49/713, C07C 303/24, C07C 305/06, C07C 213/06

(86) Numéro de dépôt international: **PCT/FR97/02062**

(87) Numéro de publication internationale: **WO 98/022435 (28.05.1998 Gazette 1998/21)**

(54) **COMPOSES UTILES POUR LA PERHALOGENOALCOYLATION, REACTIF POUR METTRE EN OEUVRE CES COMPOSES ET PROCEDE DE SYNTHESE POUR L'OBTENTION DE CES COMPOSES**

VERBINDUNGEN VERWENDBAR ZUR PERHALOALKYLIERUNG, REAGENZ FÜR IHRER DURCHFÜHRUNG UND VERFAHREN ZU IHRER HERSTELLUNG

COMPOUNDS USEFUL FOR PERHALOGENOALKYLATION, REAGENT FOR IMPLEMENTING THESE COMPOUNDS AND SYNTHESIS METHOD FOR OBTAINING THESE COMPOUNDS

(84) Etats contractants désignés:
**AT BE CH DE DK ES FI FR GB GR IE IT LI LU MC NL PT SE**

(30) Priorité: **15.11.1996 FR 9614133**
**23.05.1997 FR 9706516**
**23.05.1997 FR 9706517**

(43) Date de publication de la demande:
**06.10.1999 Bulletin 1999/40**

(73) Titulaire: **RHODIA CHIMIE**
**92408 Courbevoie Cédex (FR)**

(72) Inventeurs:
- **ROQUES, Nicolas**
  **F-69008 Lyon (FR)**
- **RUSSELL, James**
  **F-30340 Rousson (FR)**
- **LANGLOIS, Bernard**
  **F-69006 Lyon (FR)**
- **SAINT-JALMES, Laurent**
  **F-69330 Meyzieu (FR)**
- **LARGE, Sylvie**
  **F-69100 Villeurbanne (FR)**

(74) Mandataire: **Ricalens, François et al**
**Rhodia Services,**
**Direction de la Propriété Industrielle,**
**40, rue de la Haie-Coq**
**93306 Aubervilliers Cedex (FR)**

(56) Documents cités:
**EP-A- 0 614 874**
**WO-A-97/19038**
**DE-B- 1 076 113**
**US-A- 3 476 811**
**US-A- 4 359 404**

- **T. SHONO, ET AL.: "Electroorganic chemistry. 130. A novel trifluoromethylation reaction of aldehydes and ketones promoted by an electrogenerated base" JOURNAL OF ORGANIC CHEMISTRY, vol. 56, no. 1, 4 janvier 1991, WASHINGTON, DC, US, pages 2-4, XP002009863 cité dans la demande**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

EP 0 946 504 B1

Printed by Jouve, 75001 PARIS (FR)

(Cont. page suivante)

- R.W. LANG: "Fluorine-containing organozinc reagents. Part III. A new formylation reaction of fluoroalkylzinc halides" HELVETICA CHIMICA ACTA, vol. 71, no. 2, 16 mars 1988, BASEL, CH, pages 369-373, XP002054994
- M. MULLIEZ, ET AL.: "Synthèse d'alpha-hydroxysulfonates" TETRAHEDRON, vol. 49, no. 12, 19 mars 1993, OXFORD, GB, pages 2469-2476, XP002029797
- R. KRISHNAMURTI, ET AL.: "Preparation of trifluoromethyl and other perfluoroalkyl compounds with (perfluoroalkyl)trimethylsilanes" JOURNAL OF ORGANIC CHEMISTRY, vol. 56, no. 3, 1 février 1991, WASHINGTON, DC, US, pages 984-989, XP002054995
- U. HARTKOPF: "Trialkylsilyl(trifluoromethyl)diazines as tailored reagents for nucleophilic trifluoromethylation" ANGEWANDTE CHEMIE. INTERNATIONAL EDITION, vol. 21, no. 6, 1982, WEINHEIM, DE, page 443 XP002054996
- R. BARHDADI, ET AL.: "Direct electroreduction or use of an electrogenerated base: two ways for the coupling of polyhalogenated compounds with aldehydes or ketones" TETRAHEDRON, vol. 53, no. 5, 3 février 1997, OXFORD, GB, pages 1721-1728, XP002054997
- T. SHONO, ET AL.: "Novel synthesis of carbohydrates using electroreduction as key reactions" TETRAHEDRON LETTERS, vol. 23, no. 46, 1982, OXFORD, GB, pages 4801-4804, XP002054998
- M. FUJITA, ET AL.: "Fluoride ion-catalysed generation and carbonyl addition of alpha-halo carbanions derived from alpha-halo organosilicon compounds" TETRAHEDRON, vol. 44, no. 13, 1988, OXFORD, GB, pages 4135-4145, XP002054999
- J.P. IDOUX, ET AL.: "Aromatic fluoroalkoxylation via direct displaceemnt of a nitro or fluoro group" JOURNAL OF ORGANIC CHEMISTRY, vol. 50, no. 11, 31 mai 1985, WASHINGTON, DC, US, pages 1876-1878, XP002035728
- E.J.P. FEAR, ET AL.: "Organic fluorine compounds, Part V. The instability of some sodium 1:1-di-H-perfluoroalkoxides and its effect on the yields of fluoroethers" JOURNAL OF THE CHEMICAL SOCIETY, avril 1958, LETCHWORTH, GB, pages 1322-1325, XP002055004
- A.R. FERSHT: "Acyl-transfer reactions of amides and esters with alcohols and thiols. A reference system for the serine and cysteine proteinases. Concerning the N protonation of amides and amide-imidate equilibria" JOURNAL OF THE AMERICAN CHEMICAL SOCIETY, vol. 93, no. 14, 14 juillet 1971, WASHINGTON, DC, US, pages 3504-3515, XP002055000
- M.J. GREGORY, ET AL.: "Nucleophilic displacement reactions at the thiol ester bond V. Reactions of 2,2,2-trifluoroethyl thiolacetate" JOURNAL OF THE AMERICAN CHEMICAL SOCIETY, vol. 89, no. 9, 26 avril 1967, WASHINGTON, DC, US, pages 2121-2127, XP002035731

## Description

La présente invention a pour objet un procédé utile pour la perfluoroalcoylation et ainsi qu'un réactif pour mettre en oeuvre ce procédé. Elle concerne plus particulièrement un réactif et un procédé pour greffer un groupement difluorométhyle substitué sur un composé comportant au moins une fonction électrophile. Elle concerne plus particulièrement une technique pour perfluoroalcoyler différents composés par des réactions de substitution nucléophile ou d'addition typiquement réalisées par des dérivés organométalliques.
Les techniques de perfluoroalcoylation, ou les techniques équivalentes, utilisent en général des dérivés du type iodure ou bromure de perfluoroalcoyle, en présence de zinc. Cette technique est donc coûteuse, tout en nécessitant des installations de traitement des rejets métalliques qu'il convient de traiter car le zinc est un polluant important des cours d'eau. En outre les composés de type bromure de trifluorométhyle présentent un effet de serre considérable.
Les autres techniques, où le radical perfluoroalcoyle ne forme pas un intermédiaire réactif stabilisé du type organométallique, sont en général difficiles à mettre en oeuvre en raison de la très faible stabilité des anions perfluorés libres dans les milieux réactionnels. Ces derniers conduisent en général à des produits du type carbène, lesquels lorsqu'ils réagissent ont perdu un de leurs substituants. C'est pourquoi un des buts de la présente invention est de fournir un réactif qui permette une perfluoroalcoylation selon un mécanisme du type faisant intervenir carbanion, sans faire appel à des organométalliques de métaux de transition comme le zinc.
On a souvent cherché à utiliser comme source de radicaux perfluoroalcoyle, plus généralement de radicaux trifluorométhyle, des acides perfluorocarboxyliques, en mettant en oeuvre des réactions de décomposition visant à éliminer le fragment carboxylique desdits acides en libérant du bioxyde de carbone. Toutefois, les succès qui avaient été obtenus étaient très mitigés et utilisaient des systèmes catalytiques particulièrement compliqués. Les radicaux perfluoroalcoyle ou leurs équivalents engendrés par la décomposition desdits acides perfluorocarboxyliques étaient en outre instables dans le milieu réactionnel et nécessitaient l'emploi d'agents stabilisants.
Plus récemment SHONO dans un article intitulé "a novel trifluorométhylation of aldéhydes and ketones promoted by an electrogenerated base " et publié dans J. Org. Chem. 1991, 56, 2-4 a essayé de réaliser des réactions de perfluorométhylation à partir de fluoroforme et montré qu'il était très difficile d'obtenir des résultats positifs en l'absence de la base constituée de l'anion pyrolidonyle associé à un cation ammonium quaternaire et ce à la condition expresse que cette base fût engendrée par électrolyse. Cette technique utilise la voie Barbier. Au cours de cette étude comparative, en prenant comme réaction test la trifluorométhylation du benzaldéhyde selon la technique dite de Barbier (qui consiste à ajouter base et fluoroforme sur le substrat), cet auteur concluait que les résultats obtenus à partir d'autres bases donnaient des rendements nuls ou médiocres et que les réactions parasites et notamment celle de Cannizaro (dismutation du benzaldéhyde en acide benzoïque et en alcool benzylique), étaient majoritaires [mais les modes opératoires relatifs aux bases usuelles (tertiobutylate de potassium, hydrure de sodium,...) n'y sont pas décrits]. R. KRISHNAMURTI, ET AL.: "Preparation of trifluoromethyl and other perfluoroalkyl compounds with (perfluoroalkyl)trimethylsilanes" J. ORG. CHEM., vol.56 no.3, pp. 984-989 (1991), décrivent une technique de greffage d'un groupement $CF_3$ sur des cétones en utilisant le réactif de Ruppert en présence d'une base pour donner un alcoolate perfluoré.
Toutefois les techniques utilisant les bases électroengendrées décrites par cet auteur nécessitent d'une part un appareillage complexe et d'autre part un tour de main tel qu'elles sont délicates à reproduire et extrêmement difficiles à extrapoler à des échelles industrielles. Enfin l'utilisation des ammoniums quaternaires qui sont très hygroscopiques implique beaucoup de minutie.
La présente invention se propose d'obvier aux inconvénients des procédés existants en fournissant un réactif non nocif pour l'environnement et capable de conduire aux produits désirés avec un rendement satisfaisant.
Un autre but de la présente invention est de proposer des réactifs et des conditions opératoires qui pallient les inconvénients des ammoniums quaternaires et qui permettent ainsi de les utiliser.

Ces buts et d'autres qui apparaîtront par la suite sont atteints au moyen d'un réactif comportant au moins un composé de formule IV :

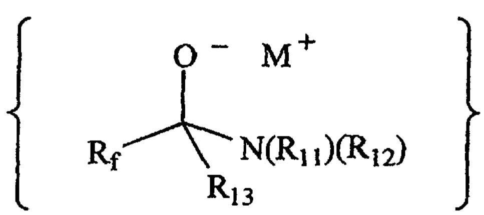

(formule IV)

où $M^+$ représente un cation monovalent avantageusement choisi parmi les alcalins, les phosphoniums et les am-

moniums quaternaires ;

où $R_{11}$, $R_{12}$ et $R_{13}$ représentent des chaînes hydrocarbonées, ces chaînes pouvant être reliées entre elles pour former un (voire plusieurs) cycle(s) ; $R_{13}$ pouvant également prendre la valeur hydrogène ;

où $R_f$ est un radical de formule :

$$GEA\text{-}(CX_2)_P\text{-} \quad (II)$$

où les X, semblables ou différents, représentent un fluor ou un radical de formule $C_nF_{2n+1}$, avec n entier au plus égal à 5, de préférence à 2, voire un chlore ;

où p représente un entier au moins égal à 1 et au plus égal à 2 ;

où GEA représente un groupe électro-attracteur dont la constante de Hammett $\sigma_p$ est au moins égale à O, avec la condition que $R_f$, $R_{13}$, $R_{11}$ et $R_{12}$ ne peuvent pas être simultanément, respectivement, trifluorométhyle, hydrogène, éthyle et méthyle ;

le nombre de carbones global dudit composé étant au plus égal à 50.

Le composé d'addition du fluoroforme sur le DMF avait peut être déjà été obtenu, mais non identifié, lors d'essai (par voie dite Grignard) servant d'exemple à la demande française (FR95/13996) non publiée lors du dépôt de la plus ancienne demande prioritaire (FR96/14133) à la présente demande.

Selon la présente invention, ledit réactif peut comporter en outre un solvant (ou mélange de solvants) polaire et peu ou non protique.

Selon un mode préféré, ledit composé de formule IV est présent à une concentration au moins égale à une millimole par litre, avantageusement à 5 millimoles par litre, de préférence 10 millimoles par litre.

Le réactif visé ci-dessus peut être obtenu par l'usage d'un autre réactif, également utile pour l'obtention de dérivé fluoré qui comporte pour addition successive ou simultanée :

une base silicophile ;
et
un matériau de formule Rf-Z avec Z choisi parmi l'hydrogène et -Si $(R')_3$, où les R' semblables ou différents sont de radicaux hydrocarbonés de 1 à environ 20 atomes de carbone, avantageusement de 1 à 10, de préférence de 1 à 5. Le nombre total de carbone de Rf-Si $(R')_3$ étant avantageusement au plus égal à 50, de préférence à 30 ;

avec la condition que lorsque Z est hydrogène, il comporte en outre un dérivé azoté trivalent persilylé, avantageusement en quantité au moins stoechiométrique.

Le dérivé azoté trivalent persilylé peut notamment être un amide persilylé dont l'anion est très basique (Par exemple le formamide persilylé) mais on préfère les amines persilylées).

Pour des raisons diverses et notamment économique le cas où Z est H est préféré. Dans ce cas le réactif comporte pour addition successive ou simultanée :

un matériau de formule RfH ;
une base silicophile et ;
un dérivé azoté trivalent persilylé.

La réaction peut se symboliser comme suit :

$$RfH + B^- + {>}N\text{- } Si\,(R')_3 \text{ --------------> } Rf^- + {>}NH + B - Si\,(R')_3$$

avec en général un équilibre :

$$Rf^- + {>}NH \text{ <--------------> } RfH + {>}N^-$$

Il est souhaitable que la base obtenue (>$N^-$)par après dessilylation dudit dérivé azoté trivalent persilylé soit au moins aussi forte que le méthylate, avantageusement que l'éthylate de sodium.

Selon la présente invention, ledit réactif peut comporter en outre un solvant (ou mélange de solvants) polaire et peu ou non protique.

Le (ou les) cation(s) associé(s) à ladite base silicophile est avantageusement choisi(s) parmi les ammoniums quaternaire, les phosphoniums quaternaires et les alcalins.

Si on désire éviter l'emploi d'ammonium quaternaires, le (ou les) cation(s) associé(s) à ladite base silicophile est de préférence choisi(s) parmi les phosphoniums quaternaires et les alcalins (avantageusement de rang au mois égal à celui du sodium).

Mais selon la présente invention la technique facilite l'emploi d'ammonium quaternaire, aussi le (ou les) cation(s) associé(s) à ladite base silicophile peuvent être choisis parmi les ammoniums quaternaires.

Lorsqu'ils sont alcalins, le (ou les) cation(s) associé(s) à ladite base silicophile est choisi(s) parmi le alcalin de nombre atomique au moins égal, de préférence supérieur, à celui du sodium.

En ce qui le concerne, l'anion silicophile des bases est avantageusement choisi parmi ceux susceptibles de former une liaison avec un silyle présentant une énergie d'au moins 110 Kcal par mole, avantageusement à environ 120, de préférence à 130. (cf R. Walsh Acc. Chem. Res.). (lorsque la base silicophile est utilisé avec un dérivé tétraédrique silylé, il est préférable de choisir comme base du fluor ou un alcoolate de pKa plus élevé que celui du composé tétraédrique).

Selon une variante avantageuse de la présente invention, l'anion des bases est choisi parmi le fluorure et ses complexes les anions alcoolates et leurs mélanges. En ce qui concerne les alcoolates on peut indiquer que, notamment en présence d'un dérivé d'azote trivalent persilylé, ils présentent un pKa au moins égal à 6, avantageusement à 8, de préférence à 9.

Ladite base silicophile est notamment choisie parmi les fluorures et les alcoolates y compris les alcoolates de formule $Rf(R_5)(R_6)C-O^-$ où Rf a déjà été défini et où $R_5$ et $R_6$ sont choisis parmi l'hydrogène et des restes hydrocarbonés et ne représentent avantageusement pas de groupe électroattracteur fort, c'est-à-dire que l'on choisira de préférence les groupes fonctionnels dont la constante de Hammett $\sigma_p$ est au plus égale à 0,2, plus préférentiellement à 0,1.

L'anion tétraédrique obtenu avec les composés de type carbonylé est ainsi en général lui-même silicophile. De ce fait, dans ce cas, la base peut être utilisée en quantité catalytique (avantageusement 1/50 à 1/2, de préférence de 1/20 à 1/3 de la QS (c'est-à-dire Quantité Stoechiométrique)). Il est à souligner que cette technique pallie certains inconvénients de l'utilisation des ammoniums quaternaires et permet d'éviter l'appel aux bases électroengendrées. Elle conduit directement à des composés au moins en partie silylés.

Ainsi ce procédé consiste à faire réagir un composé de formule >N-Si $(R')_3$ avec une base (dont l'anion est) silicophile, où les R' semblables ou différents sont de radicaux hydrocarbonés de 1 à environ 20 atomes de carbone, avantageusement de 1 à 10, de préférence de 1 à 5. Le nombre total de carbone de >N-Si $(R')_3$ étant avantageusement au plus égal à 50, de préférence à 30. Les radicaux silyles auxquels il est fait référence dans la présente description présentent avantageusement les mêmes caractéristiques.

Avantageusement ledit anion silicophile est choisi parmi le fluorure, ses complexes (par exemple TBAT), les anions oxygénés [avantageusement alcoolate et notamment perfluorocarbinolate (perfluoro- au sens de Rf)] et leurs mélanges.

Ledit dérivé azoté trivalent persilylé présente avantageusement une masse moléculaire d'au plus environ 1000. Ainsi il possède au plus environ 50 atomes de carbone et est de préférence choisi parmi l'ammoniac persylilé et les amines primaires persilylés et leurs mélanges. Les différents groupes silyles peuvent être différents ou semblables, ce dernier cas étant toutefois plus simple et plus économique.

Selon une mise en oeuvre particulièrement avantageuse de la présente invention, l'anion des bases et ledit dérivé azoté trivalent persilylé sont choisis de manière que l'anion silylé présente un point d'ébullition sous pression atmosphérique au plus égal à environ 100°C, avantageusement à 50°C. Cette propriété permet de déplacer plus facilement l'équilibre en éliminant progressivement B-Si $(R')_3$ [et même rend possible son élimination au fur et à mesure de sa formation éventuellement sous pression réduite] permettant ainsi l'utilisation de base relativement faible.

Pour ce faire il est conseillé de faire en sorte que ledit réactif comporte une phase gazeuse dont la pression partielle en l'anion silylé est inférieur à la pression saturante dans les conditions opératoires à celle dudit anion silylé.

Ledit réactif comporte avantageusement en outre au moins un composé (qui peut être aussi le solvant) porteur d'au moins une double liaison de type >C=Y où Y représente un azote avantageusement substitué ou plutôt un atome de chalcogène. Ces composés sont soit un vecteur de l'entité désignée par $R_f^-$ soit forme avec $R_f^-$ un intermédiaire réactionnel.

Dans ce cas le réactif ainsi obtenu par mise en contact dans un milieu polaire et peu ou non protique, d'un matériau de formule RfH et d'une base avec un substrat porteur d'au moins une double liaison de type >C=Y où Y représente un azote avantageusement substitué ou plus préférentiellement un atome de chalcogène (avantageusement soufre ou plus généralement oxygène) est assez stable et permet soit de constituer un intermédiaire réactionnel de qualité

soit un réactif perfluoroalcoylant.
Les conditions d'utilisation des réactifs ci-dessus sont sensiblement les mêmes et sont détaillées ci-dessous.
Dans la présente description, par H-Rf on entend des radicaux de formule :

$$H\text{-}(CX_2)_p\text{-}GEA \qquad (II)$$

où les X semblables ou différents représentent, un fluor ou un radical de formule $C_nF_{2n+1}$ avec n entier au plus égal à 5 de préférence à 2, voire un chlore ;
où p représente un entier au moins égal à 1 et au plus égal à 2 ;
où GEA représente un groupe électroattracteur dont les éventuelles fonctions sont inertes dans les conditions de la réaction, avantageusement fluor ou un reste perfluoré de formule $C_nF_{2n+1}$ avec n entier au plus égal à 8, avantageusement à 5.

Avantageusement X ne peut être qu'une fois chlore sur un même carbone. Le cas où le carbone portant l'atome d'hydrogène, présente deux X différents de chlore est particulièrement intéressant.
Il est également souhaitable que parmi les X et GEA au moins un avantageusement 2 soient des atomes (de chlore ou de fluor).
Le nombre total de carbone de Rf est avantageusement compris entre 1 et 15, de préférence entre 1 et 10.
Dans le matériau RfH du réactif de l'invention, l'entité GEA qui exerce un effet électroattracteur sur l'atome de carbone difluoré est de préférence choisie parmi les groupes fonctionnels dont la constante de Hammett $\sigma_p$ est au moins égale à 0,1. Il est en outre préférable que la composante inductive de $\sigma_p$, $\sigma_i$, soit au moins égale à 0,2, avantageusement à 0,3. A cet égard, on se référera à l'ouvrage de March, "Advanced Organic Chemistry", troisième édition, John Wiley and Son, pages 242 à 250, et notamment au tableau 4 de cette section.
Plus particulièrement, l'entité électroattractrice peut être choisie parmi les atomes d'halogène, de préférence légers, notamment de chlore et de fluor. Le matériau RfH correspondant est, quand p est égal à 1, un Halogénoforme.
GEA peut également être avantageusement choisie parmi les groupements nitriles, carbonylés, sulfonés et perfluoroalcoylés.
Des matériaux préférés de formule RfH de ce type qui peuvent être utilisés répondent à la formule R - G - $CF._2$- H

- où G représente un groupement divalent de formule -Z-G'- dans lequel
  - ♦ le divalent Z représente une liaison simple, un atome de chalcogène, un reste divalent -Y(R')- où R' est un radical hydrocarboné d'au plus dix atomes, avantageusement d'au plus six atomes avantageusement d'au plus deux atomes de carbone et où Y est un atome métalloïde de la colonne V (azote, phosphore,....) ;
  - ♦ G' représente >C=O, >S=O, $-SO_2-$, ou - (CF.2) $_n$- avec n entier supérieur ou égal à 1 ;
- et où R représente un résidu organique ou minéral indifférent, de préférence un radical organique tel que aryle, alcoyle, y compris aralcoyle, éventuellement substitué. R peut également représenter un support solide minéral ou organique, tel qu'une résine ;
- ou bien l'ensemble R - G représente un groupe nitrile, ester, amide (avantageusement non porteur d'hydrogène), y compris sulfamide.

Dans le cas où G représente un groupe perfluoroalcoylène - $(CF._2)_n$ -, n est avantageusement compris entre 1 et 10, de préférence entre 1 et 5. Toujours dans ce cas, R peut également représenter un atome d'halogène, notamment le fluor.
Ainsi, selon une variante avantageuse de la présente invention, ledit matériau de formule $R_fH$ répond à la formule II où GEA représente un groupe électroattracteur de formule III :

$$R\text{-}C_nX'_{2n}\text{-} \qquad (III)$$

Où n est un entier au plus égal à 5,
où R est choisi parmi l'hydrogène, un radical hydrocarboné, tel que les aryles et les alcoyles de 1 à 10 atomes de carbone, et les halogènes légers (chlore ou fluor, avantageusement fluor) ;
où les X', semblables ou différents représentent un halogène léger (chlore ou fluor, avantageusement fluor) ou un radical de formule $C_mF_{2m+1}$ avec m entier au plus égal à 5 de préférence à 2.

Lorsque R représente un hydrogène, la réaction est plus complexe, ledit matériau pouvant réagir par plusieurs bouts ; et les rapports entre les réactifs doivent tenir compte de cette réactivité dans la stoechiométrie. Cette polyvalence des matériaux peut être un inconvénient et de ce fait la valeur hydrogène pour R n'est pas communément souhaitable.
Il est souhaitable que au moins trois quarts, avantageusement au moins neuf dixièmes, de préférence la totalité, éventuellement moins un, des X et des X' soient des fluors ou des perfluoroalcoyles (stricto sensu soit de formule générale de type $C\nu F_2\nu_{+1}$)
Selon la présente invention ledit substrat est avantageusement un composé de type:

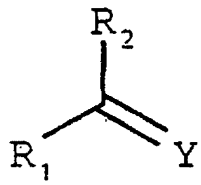
(formule I)

où Y à déjà été défini et
où $R_1$ est un radical choisi parmi l'hydrogène, les radicaux hydrocarbonés tels que aryle (y compris alcoylaryle), alcoyle (y compris aralcoyle et cycloalcoyle)
où $R_2$ est un radical choisi parmi les radicaux hydrocarbonés tels que aryle (y compris alcoylaryle), alcoyle (y compris aralcoyle et cycloalcoyle), parmi les fonctions amines avantageusement persubstituées (c'est-à-dire dont l'azote ne porte plus d'hydrogène), les fonctions acyloxyles, les fonctions hydrocarbyloxyles, avec la condition que lorsque R1 est hydrogène R2 n'est ni phényle ni diméthyl amino.

Ces chaînes $R_1$ et $R_2$ peuvent être reliées entre elles pour former un (voire plusieurs) cycle(s). Avantageusement les substituants sont tels que la somme des carbones présents dans la molécule est au plus égale à environ 50, de préférence à 30. Il est souhaitable que l'un des, avantageusement les deux, $R_1$ et $R_2$ ne soit pas encombrant, c'est-à-dire l'atome reliant ledit radical à la dite fonction >C=Y ne porte lui-même qu'au plus deux chaînes (radical secondaire), de préférence qu'au plus une chaîne (radical primaire).
$R_1$ et $R_2$ peuvent être un radical amine secondaire (dialcoylamino) et dans ce cas l'on observe une augmentation significative de l'espérance de vie de l'entité désignée par $R_f^-$. Mais le complexe tétraédrique est trop fugitif pour servir de réactif en temps que tel et doit être fait in situ. Ils n'existent pas, non plus que les autres complexes tétraédriques de type hémiaminal, ou du moins n'ont pu être détectés, en présence de substrat cétonique ou aldéhydique.
Les complexes tétraèdriques y compris ceux correspondant au DMF peuvent également être obtenus par l'action d'une sur un radical de formule Rf-($R_1$)($R_2$)-C-O-Z' où Z' est choisi parmi l'hydrogène (la formule correspond alors à un hémiaminal lorsque, comme cela est préféré, $R_2$ est un radical choisi parmi les fonctions amines) et -Si $(R')_3$, où les R' semblables ou différents sont de radicaux hydrocarbonés de 1 à environ 20 atomes de carbone, avantageusement de 1 à 10, de préférence de 1 à 5. Le nombre total de carbone de Rf-Si $(R')_3$ étant avantageusement au plus égal à 50, de préférence à 30. Ladite base est avantageusement silicophile surtout dans le cas ou Z' est silyle.
Lorsque Z est égal à H et forme un hémiaminal, ce dernier est avantageusement obtenu par action d'une amine secondaire sur l'aldhéhyde de formule $R_f$ - CHO avec déshydratation.
Ladite déshydratation est de préférence réalysée par distillation (hétéro)azéotropique de l'eau (diluant (Par exemple toluène) ou amine elle-même [Par exemple cas de la dibutylamine]). Elle peut aussi être menée avec un dehydratant.
Egalement, bien qu'il ait été de nombreuses fois préconisé d'utiliser avec les agents de perfluoroalcoylation, des éléments de la colonne VIII de la classification périodique des éléments, pour favoriser certains substrats et favoriser certains types de réaction, cela ne s'est pas révélé particulièrement faste pour la réaction. visée ci-dessus. C'est pourquoi il est préférable d'utiliser des réactifs ne contenant pas de métaux de la colonne VIII, notamment des métaux de la mine du platine qui est le groupe constitué du platine, de l'osmium, de l'iridium, du palladium, du rhodium et du ruthénium.
Dans la présente description, il est fait référence au supplément au bulletin de la Société Chimique de France numéro 1, janvier 1966, où une classification périodique des éléments a été publiée.
Ainsi il est préférable que la teneur en métaux de la mine du platine, voire en métaux de la colonne VIII, soit inférieure à 100 ppm, avantageusement à 10 ppm, de préférence 1 ppm. Ces valeurs s'entendent par rapport à l'acide la base de départ et sont exprimées en moles.
D'une manière plus générale et plus empirique, on peut indiquer qu'il est souhaitable que ces deux catégories de métaux, à savoir les éléments de transition à deux états de valence et les éléments de la colonne VIII, soient chacune

présente dans le réactif à un niveau de concentration globale au plus égal à 1000 ppm molaires, de préférence à 10 ppm molaires.

On notera que les différents métaux présents à un tel niveau de concentration globale sont en quantité extrêmement faible et, à cet égard, ils ne jouent aucun rôle catalytique. Leur présence n'améliore pas la cinétique de la réaction, voire lui est néfaste lorsqu'ils sont présents en trop grande quantité.

Sauf en présence d'agent silylant où il jouent le rote de base (voir ci-dessus), l'utilisation, en plus des composants de réactifs précités, de fluorure de métal alcalin ou de fluorure de phosphonium quaternaire [voire d'ammonium quaternaire si l'on respecte les contraintes que ce type de composé engendre], habituellement présents dans les systèmes réactifs utilisant des carboxylates fluorés, ne s'est pas révélée néfaste, mais elle s'est révélée en général de peu d'intérêt, notamment en raison du fait qu'elle produit des effluents salins difficiles à traiter. C'est la raison pour laquelle il est préférable de limiter leur teneur, en particulier leur teneur initiale. Ainsi il est préférable que la teneur en fluorure, que l'on qualifie de ionique, c'est-à-dire susceptible d'être ionisé dans le milieu polarisant du réactif, soit au plus égale à la concentration molaire initiale en ledit matériau RfH, avantageusement à la moitié, de préférence au quart.

Lorsque le substrat est sensible à la dégradation par base, il convient d'en limiter la quantité et surtout l'excès ; ainsi dans le cas où les substrats sont susceptibles de se dismuter, comme c'est le cas des aldéhydes pouvant donner lieu à des réactions de Cannizaro et/ou de Tishchenko, ou des réactions de crotonisation, il convient de limiter la quantité de base à 4/3, avantageusement à 5/4, de préférence à 1,1 la Q.S. (c'est-à-dire Quantité Stoechiométrique) par rapport au substrat.

L'utilisation de substrat non-sensible à la dégradation par base, permet ainsi d'utiliser de larges excès de base et donc de réactif. Alors un excès de 20 à 300 % est possible ; mais il est préférable de le limiter pour des questions de coût, en général, à une valeur d'environ 100 %. Bien entendu les mêmes valeurs sont applicables que lorsque le substrat est sensible aux bases.

Lorsque le substrat joue aussi un rôle de solvant, on peut alors utiliser des quantités de base bien inférieure (Cf. infra).

La réaction est en général menée à une température comprise entre la température de fusion du milieu et la température d'ébullition dans les conditions de pression de la réaction.

Plus spécifiquement, la réaction est menée en phase liquide, à une température comprise entre environ -100°C et 0°C, avantageusement entre environ - 60°C et - 10°C. Lorsque $R_fH$ est très volatil il est préférable pour s'assurer qu'il n'y ait pas d'évaporation ; pour ce faire il convient soit d'éviter que l'écart entre la température de réaction et le point d'ébullition soit trop important, plus précisément, il est souhaitable de travailler à une température qui ne soit pas supérieure au point d'ébullition (sous pression atmosphérique) de plus 100°C (deux chiffres significatifs), avantageusement de plus 80°C ; soit de travailler en enceinte fermée, soit d'opérer sous pression partielle élevée dudit $R_fH$. Soit de travailler en technique Grignard. Il est possible et même avantageux de combiner au moins deux des mesures ci-dessus.

Enfin quand la formation de carbène est favorisée par la température cette formation de carbène est corrélée à un dégagement d'acide halohydrique ce qui favorise les réactions parasites. Aussi est-il préférable d'éviter de travailler à des températures au plus égales à l'ambiante (20°C), de préférence à une température d'au plus -20°C, lorsqu'il y a un tel risque (essentiellement lorsque le nombre de carbone de RfH est égal à 1 ou lorsque GEA et/ou X est un chlore).

Lorsque le substrat est sensible à la dégradation basique il est également préférable d'opérer à une température au plus égale à l'ambiante. S'il y a risque double de formation de carbène et sensibilité au base du substrat il est alors préférable de ne pas dépasser environ -20° C .

Selon une mise en oeuvre particulièrement avantageuse de la présente invention, la réaction est menée de manière à introduire en premier dans le milieu soit le matériau RfH, soit la base, et enfin le substrat. En d'autre terme de constituer un réactif à partir du matériau RfH, de la base et le cas échéant du solvant et/ou du diluant, puis de faire agir ce réactif sur le substrat. Cette variante sera désignée ci-après sous l'expression de variante Grignard.

La réaction est menée de manière à introduire le dernier composant du réactif progressivement et avantageusement sur une période de temps comprise entre 5 et 300 minutes.

La réaction est menée de manière à introduire ladite base progressivement et avantageusement sur une période de temps comprise entre 5 et 300 minutes.

Ainsi, au cours de l'étude qui a mené à la présente invention, Il a été montré que le réactif conduisait à une nouvelle espèce

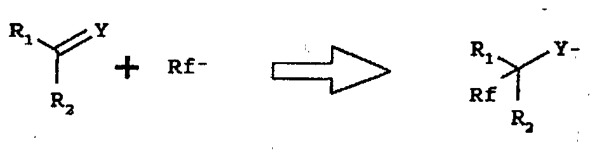

(formule V)

Cette espèce,

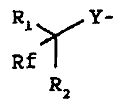

$M^+$ n'a été décrite nulle part à la connaissance de la Demanderesse. Aussi une caractéristique majeure du réactif selon la présente invention est la présence de ladite espèce (ou de plusieurs) dans le réactif ; avantageusement à des concentrations qui seront exposées ci-après pour les dérivés d'amides.

Aussi la présente invention vise les composés de formule IV :

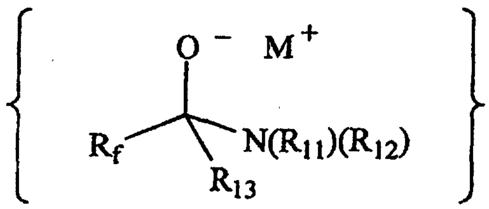

**(formule IV)**

Dans cette formule $M^+$ représente un cation avantageusement monovalent et correspondant aux bases spécifiées dans la présente description ; avantageusement les alcalins et les phosphoniums. Les préférences pour les Rf et $R_{13}$ sont exposées ci-après pour les dérivés d'amides.

Ce composé intermédiaire tétraédrique peut servir de réactif de perfluoroalcoylation(s), comme décrit ci-dessus, mais aussi peut constituer un intermédiaire réactionnel conduisant à d'autres composés intéressants que ceux issus de l'hydrolyse. La forme tétraédrique peut notamment être bloquée par les agents (essentiellement électrophiles) connus pour s'additionner aux alcoolates, En particulier pour donner des dérivés O-silylés, O-acylés, (bi)sulfitique. Ces dérivés bloqués constituent des réactifs et des intermédiaires de synthèse particulièrement intéressants.

Lorsqu'il est utilisé comme réactif de perfluoroalcoylation, il est préférable que les substituants $R_1$ ,$R_2$ et celui éventuel de Y soient de faible taille, c'est-à-dire que lorsqu'ils sont alcoyles leur nombre de carbones soit avantageusement au plus égal à 6, avantageusement à 3, de préférence méthyles ; lorsqu'ils sont aryles, avantageusement phényles (substitués ou non), il est préférable que leur nombre de carbones soit avantageusement au plus égal à 10, avantageusement à 8. Il est préférable que globalement les $R_1$ ,$R_2$ et Y présente un nombre de carbones au plus égal à 15, avantageusement à 12, de préférence à 8.

Lorsqu'il n'est pas utilisé comme réactif de perfluoroalcoylation, mais comme intermédiaire de synthèse, les $R_1$ ,$R_2$ et Y peuvent être de taille plus élevée (sous réserve qu'il soit au moins partiellement, de préférence totalement soluble dans le milieu), le nombre global de carbone peut alors atteindre environ 50. Cependant il est préférable que l'on ne dépasse pas environ 30 atomes de carbone.

Lorsqu'ils sont employés, les amides utilisés jouent un rôle dans la formation du réactif. En effet on a pu mettre en évidence que le réactif formé dans les amides (surtout lorsqu'ils répondent au précurseur de la formule du composé tétraédrique ci-après) était un réactif dont l'espèce réactive était le composé d'addition du $CF._3^-$ sur le carbone de la fonction carbonyle, l'oxygène de cette dernière fonction devenant anionique.

C'est ce composé qui joue le rôle de vecteur de $CF._3$- ou plus exactement de $Rf^-$. selon la présente invention, les composés de formule V peuvent être également obtenus par les voies suivantes :

addition d'une amine sur l'hydrate de fluoral ou le fluoral (dans les deux équations de réactions suivantes, $CF_3$ est le paradigme de Rf):

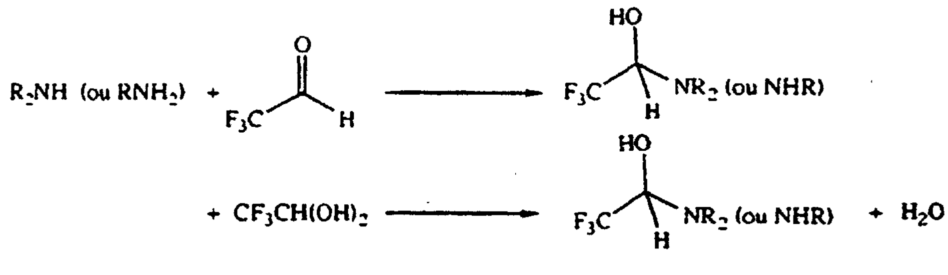

suivi d'une anionisation au moyen d'une base, par exemple du type de celle visée dans le présente demande. Mais ces bases peuvent être des bases relativement faibles.

Les conditions opératoires sont avantageusement les mêmes que celles de la technique qui vient d'être détaillée plus haut mais la présence d'amide, quoique souhaitable, n'est pas indispensable.

Les amines peuvent être de type $R_2NH$ ou $RNH_2$ (R= alcoyle ou aryle simple et correspondant à $R_{11}$ et/ou $R_{12}$ de la formule IV), cette méthode n'a jamais été décrite.

Selon une autre voie possible on peut utiliser les dérivés de type Rf- Si-$(R)_3$ en présence d'une base dont l'anion peut être silicophile. L'anion tétraédrique obtenu est lui-même silicophile la base peut être en quantité catalytique (avantageusement 1/50 à 1/2, de préférence de 1/20 à 1/3 de la QS (c'est-à-dire Quantité stoechiométrique)). La réaction peut s'écrire en prenant comme modèle $CF_3SiEt_3$ et le formamide et comme silicophile un fluorure (d'ammonium quaternaire). Il est à noter que cette technique pallie certains inconvénients de l'utilisation de ammonium quaternaires.

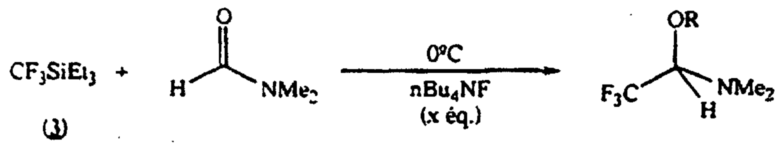


(3)

(4a) (R= nBu4N)
(4b) (R= SiEt3)

Ainsi ce procédé consiste à faire réagir un composé de formule Rf-Si $(R')_3$ avec une base dont l'anion est silicophile, où les R' semblables ou différents sont de radicaux hydrocarbonés de 1 à environ 20 atomes de carbone, avantageusement de 1 à 10, de préférence de 1 à 5. Le nombre total de carbone de Rf-Si $(R')_3$ étant avantageusement au plus égal à 50, de préférence à 30.

Par silicophile on entend les bases qui sont susceptibles de former une liaison avec un silyle présentant une énergie d'au moins 110 Kcal par mole, avantageusement à environ 120, de préférence à 130. (cf R. Walsh Acc. Chem. Res.).

Selon une mise en oeuvre avantageuse de la présente invention, le composé de formule V et notamment de formule IV peut être obtenu par réaction d'une base (avantageusement silicophile dans le cas où $R_{20}$ est silyle) sur un composé de formule $R_1$ $R_2$ Rf C-Y-$R_{20}$

où $R_{20}$ est un radical avantageusement d'au plus environ 10 carbones, choisi parmi les silyles, les acyles ;

où Y représente un azote, avantageusement substitué, ou un atome de chalcogène ;

où $R_1$ est un radical choisi parmi l'hydrogène, les radicaux hydrocarbonés tels que aryle, alcoyle;

où $R_2$ est un radical choisi parmi les radicaux hydrocarbonés tel que aryle, alcoyle, parmi les fonctions amines avantageusement persubstituées, les fonctions acyloxyles, les fonctions hydrocarbyloxyles.

Aussi une caractéristique importante de ce nouveau réactif est la présence de cette espèce dans le réactif. Aussi la présente invention vise en particulier les composés de formule (IV) Rf-C[O-$(M^+)$][$R_{13}$][N($R_{11}$)($R_{12}$)] et les réactifs en contenant au moins un.

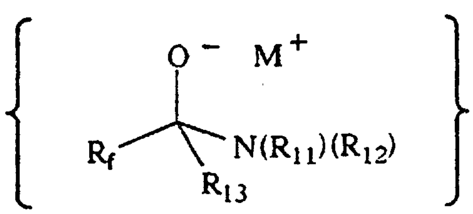
(formule IV)

Bien sûr, la formule ci-dessus vise aussi l'autre énantiomère.

Cet intermédiaire est identifiable par RMN du fluor (dans le cas du diméthylformamide, δ de 1 ppm environ [doublet difficile à résoudre] par rapport à $HCF_3$).

Dans cette formule $M^+$ représente un cation avantageusement monovalent et correspondant aux bases spécifiées dans la présente description; avantageusement les alcalins et les phosphoniums.

Rf a déjà été défini ci dessus, $R_{11}$ ,$R_{12}$ et $R_{13}$ représentent des chaînes hydrocarbonées aryle, y compris alcoylaryle, alcoyle, y compris aralcoyle et cycloalcoyle, ces chaînes peuvent être reliées entre elles pour former un (voire plusieurs) cycle(s). En ce qui concerne $R_{13}$ présente une valeur de constante de Hammett inférieure en valeur absolue à 0,2, de préférence à 0,1.

Toutefois $R_{13}$ peut aussi prendre la valeur hydrogène et c'est là sa valeur préférée. Une autre valeur satisfaisante de $R_{13}$ est la valeur aryle avantageusement dont la constante de Hammett est inférieure en valeur absolue à 0,2, de préférence à 0,1.

Cet intermédiaire présente une bonne stabilité, notamment aux basses températures (Par exemple -10, avantageusement -20, de préférence -30°C).
Ainsi selon la présente invention, vise un réactif du type précédent qui comporte au moins un composé de formule IV à une concentration au moins égale à une millimole par litre, avantageusement à 5 millimoles par litre, de préférence 10 millimoles par litre.
Cet intermédiaire peut servir de réactif de perfluoroalcoylation(s), comme décrit ci-dessous, mais aussi peut constituer un intermédiaire réactionnel conduisant des composés intéressants. En particulier aldéhyde, dérivé O-silylé, dérivé (bi)sulfitique,.O-acylé.
Lorsqu'il est utilisé comme réactif de perfluoroalcoylation, il est préférable que les $R_{11}$ ,$R_{12}$ et $R_{13}$ soient de faible taille, c'est-à-dire que lorsqu'ils sont alcoyles leur nombre de carbones soit avantageusement au plus égal à 6, avantageusement à 3, de préférence méthyles; lorsqu'ils sont aryles, avantageusement phényles (substitués ou non), il est préférable que leur nombre de carbones soit avantageusement au plus égal à 10, avantageusement à 8. Il est préférable que globalement les $R_{11}$ ,$R_{12}$ et $R_{13}$ présente un nombre de carbones au plus égal à 15, avantageusement à 12, de préférence à 8.
Lorsqu'il n'est pas utilisé comme réactif de perfluoroalcoylation, mais comme intermédiaire de synthèse, les $R_{11}$ ,$R_{12}$ et $R_{13}$ peuvent être de taille plus élevée (sous réserve qu'il soit soluble dans le milieu), le nombre global de carbone peut alors atteindre environ 50. Cependant il est préférable que l'on ne dépasse pas environ 30 atomes de carbone.
Il est donc très recommandable d'utiliser, seul(s) ou en mélange (éventuellement avec d'autres amides), les amides de formule $R_{13}$-CO-N($R_{11}$)($R_{12}$), le rapport préconisé entre ces amides et la base utilisée étant alors d'être au moins égal à 1, avantageusement à 2, de préférence à 5. Il n'y a pas de limite supérieure, sinon qu'il(s) constitue(nt) la totalité du solvant polaire. Quand ces amides sont utilisés comme solvants le plus souvent dans les tests effectués (sans que cela soit nécessairement un optimum) la teneur en ces amides par rapport à la somme des solvants polaires est comprise entre environ 40 et 80 %.

Si l'on s'intéresse aux solvants, comme solvant aprotique polaire, il est ainsi préférable d'utiliser ceux qui aient un moment dipolaire significatif. Ainsi, sa constante diélectrique relative ε est avantageusement au moins égale à environ 5. De préférence l'ε est inférieur ou égal à 50 (les zéros de position ne sont pas considérés comme des chiffres significatifs dans la présente description à moins qu'il en soit précisé autrement) et supérieur ou égal à 5.

On préfère en outre que les solvants de l'invention soient susceptibles de bien solvater les cations (ce qui est souvent rélié à la basicité du solvant) , ce qui peut être codifié par l'indice donneur D de ces solvants. Il est ainsi préférable que l'indice donneur D de ces solvants soit compris entre 10 et 30.

Pour ce qui des exigences concernant la basicité du solvant organique à mettre en oeuvre, on rappellera que "l'indice donneur", le "nombre donneur" ou "donor number", est parfois désigné de manière abrégée "DN", donne une indication sur le caractère nucléophile du solvant et révèle son aptitude à donner son doublet.

Dans l'ouvrage de Christian REINHARDT, [Solvents and Solvent Effects in Organic Chemistry - VCH p.19 (1988)], on trouve la définition du "donor number" qui est défini comme le négatif (-ΔH) de l'enthalpie (Kcal/mol) de l'interaction entre le solvant et le pentachlorure d'antimoine, dans une solution diluée de dichloroéthane.

Selon la présente invention, il est préférable que le réactif ne présente pas d'hydrogène acide sur le ou les solvants polaires qu'il utilise. En particulier lorsque le caractère polaire du ou des solvants est obtenu par la présence de groupes électroattracteurs, il est souhaitable qu'il n'y ait pas d'hydrogène en alpha de la fonction électroattractrice.

De manière plus générale, il est préférable que le pKa correspondant à la première acidité du solvant soit au moins égal à environ 20 ("environ" soulignant que seul le premier chiffre est significatif), avantageusement au moins égal à 25, de préférence compris entre 25 et 35.

Il est préférable que les composants deu milieur réactionnel, notamment ladite base et en particulier ledit matériau $R_fH$, soit au moins partiellement, de préférence complètement, soluble dans le milieu constituant le réactif.

Les solvants donnant de bons résultats peuvent être notamment des solvants de type amide. Parmi les amides, on comprend aussi les amides à caractère particulier comme les urées tétrasubstituées et les lactames monosubstitués. Les amides sont, de préférence, substitués (disubstitués pour les amides ordinaires). On peut citer par exemple les dérivés de pyrrolidone, tels que la N-méthylpyrrolidone, ou encore le N,N-diméthylformamide, ou le N,N-diméthyllacétamide.

Une autre catégorie particulièrement intéressante de solvants est constituée par les éthers, qu'il soient symétriques ou non symétriques, qu'ils soient ouverts ou non. Dans la catégorie des éthers doivent être incorporés les différents dérivés des éthers de glycol tels que les différents glymes, le diglyme par exemple.

Ainsi les solvants les plus adéquats, en raison de leurs prix et de leurs propriétés, sont avantageusement choisis parmi les éthers, notamment cycliques, tel le THF ou polyfonctionnels tels les glymes, ceux des amides qui, tels le DMF ou les DAAU (N,N'DiAlcoylAlcoylèneUrée) tels que la DMEU (N,N'DiMéthylEthylène-Urée) ou la DMPU (N,N'DiMéthylPropylèneUrée) n'ont pas d'hydrogène acide. Les hétérocycles à caractère basique tels la pyridine, peuvent être utilisés sans qu'ils constituent une classe de solvants préférés.

Parmi les diluants, on peut citer les hydrocarbures aliphatiques ou aromatiques, tels les alcanes ou les dérivés

aryliques. Mention doit être faite des arylméthanes qui peuvent à la fois servir de diluant, (car inerte dans les conditions de la réaction) et de sources de base lorsque cette dernière est faite préalablement in situ.

L'utilisation de ce réactif comme agent perhalogénoalcoylant, se fait simplement en ajoutant ledit réactif au substrat cible considéré ou vice-versa, les dérivés d'amide constituent à ce jour les meilleurs réactifs (formule IV).

Les substrats cibles sont avantageusement choisis parmi ceux porteurs d'au moins une fonction électrophile par addition. En d'autres termes que la réaction se fasse, en tout cas à titre transitoire, par addition sur une fonction présentant une double liaison (naturellement y compris celle de type donneur accepteur) ou un doublet appartenant à un métalloïde de période de rang au moins égale à 3.

Ainsi, selon une mise en oeuvre particulièrement avantageuse de la présente invention, une telle fonction électrophile par addition, est choisie parmi les fonctions carbonyle, thiocarbonyle(>C=S), éventuellement conjuguées avec une ou plusieurs liaisons de type éthylénique, les chalcogènures (dont le chalcogène est de rang atomique au moins égal à celui du soufre) portant un bon groupe partant (voir ci-dessus) et notamment les dichalcogènures (dont les chalcogènes sont des rangs atomique au moins égal à celui du soufre). Il est alors à noter que la réaction est défavorisée dans le cas où le chalcogène est porteur d'un radical encombré (radical secondaire et surtout tertiaire) et/où dont le carbocation est stabilisé (radical de type benzylique ou tertioalcoyle).

Ainsi, le réactif réagit également avantageusement sur un composé choisi parmi les composés carbonylés de type cétone, aldéhyde, ester activé ou (voire halogénure d'acide), en réalisant une addition sur la fonction carbonyle. Le produit de la réaction est un alcool(ate) dont l'atome de carbone porteur de la fonction hydroxyle est substitué par un groupement difluorométhyle substitué. Ces alcoolates transitoires, après hydrolyse (en général acide), donnent le composé de substitution ou d'addition. Le cas des amides est développé dans le passage relatif à l'intermédiaire tétraédrique.

Lorsque la fonction électrophile du substrat risque de donner des réactions de type transestérification avec la base, il est alors souhaitable que la basicité du groupe partant soit similaire ou supérieure à celle de la base mise initialement comme réactif.

La réaction est avantageusement menée à une température similaire et dans des conditions similaires à celles de la formation du réactif.

La présente invention vise aussi les composés de formule:

$$R_1\ R_2\ Rf\ C\text{-}Y\text{-}R_{20}$$

où $R_{20}$ est un radical avantageusement d'au plus environ 10 carbones, choisi parmi les alcoyles, les silyles, les acyles, celui correspondant au combinaison bisulfitique ;

où Y représente un azote, avantageusement substitué, ou un atome de chalcogène ;

où $R_1$ est un radical choisi parmi l'hydrogène, les radicaux hydrocarbonés tel que aryle, alcoyle ;

où $R_2$ est un radical choisi parmi l'hydrogène, les radicaux hydrocarbonés tel que aryle, alcoyle, parmi les fonctions amines avantageusement persubstituées, les fonctions acyloxyles, les fonctions hydrocarbyloxyles, avec la condition que lorsque $R_1$ est hydrogène, que $R_2$ est phényle ou diméthylamino et que Rf ne comporte qu'un carbone, $R_2$ ne peut être silyle de moins de 10 carbones.

Ou plus restrictivement, avec la condition que lorsque $R_1$ est hydrogène, $R_2$ n'est ni phényle ni diméthyl amino ou avec la condition lorsque Rf ne comporte qu'un carbone $R_2$ ne peut être silyle de moins de 10 carbones.

Les composés ci-dessus (y compris ceux auxquels il a été avantageusement renoncé) sont aussi utiles (lorsque $R_{20}O^-$ esun bon groupe partant) pour former un sel de Vilsmeyer ($CF_3CH=N^+Me_2$ en général avec comme contre anion le dit groupe partant $R_{20}O^-$)

Dans la présente description, est considéré comme comme bon groupe partant les composé de type pseudohalogène, c'est-à-dire un radical (en général ce radical possède un chalcolgène léger (soufre ou de préférence oxygène) par lequel il est relié au reste de la molécule) qui en partant constitue un anion dont l'acide associé présente une acidité (mesurée par la constante de Hammett) au moins égale à celle de l'acide acétique. Parmi les pseudohalogènes typiques on peut citer les radicaux acyloxyles correspondant aux acides perhalogénés en alpha de la fonction acyloxyle, tels le trifluoroacétyloxyle ($CF_3$ - CO-O-), et surtout les radicaux sulfonyloxyles, surtout ceux dont le carbone porteur du soufre est perfluoré dont le paradigme est le trifluorométhylsulfonyloxyle ($CF_3$ - $SO_2$-O-).

Pour la présente invention, on préfère ceux des pseudohalogènes qui en partant présentent une acidité au moins égale à celle des acides sulfoniques, tel le tosylique (paradigme des acides arylsulfoniques) ou le mésylique (paradigme des acides alcoylsulfoniques).

Mode opératoire à caractère général utilisé notament pour les exemples

**ABREVIATIONS**

| | |
|---|---|
| cata. : | Catalytique |
| $CDCl_3$ : | Chloroforme deutérié |
| $CFCl_3$ : | Trichlorofluorométhane |
| $ClSiMe_3$ : | Chlorure de triméthylsilane |
| CsF : | Fluorure de césium |
| DMEU : | N,N'-diméthyléthylèneurée |
| DMF : | Diméthylformamide |
| DMPU : | N,N'-diméthylpropylèneurée |
| eq : | Equivalent |
| $HCF_3$ : | Fluoroforme |
| HCl : | Acide chlorhydrique |
| HMDZ : | Hexaméthyldisilazane |
| $LiN(SiMe_3)_2$ : | Bis-(triméthylsilyl)amidure de lithium |
| M : | Molarité |
| $Me_4NF$ : | Fluorure de tétraméthylammonium |
| mmol : | Millimole |
| $N(SiMe_3)_3$ : | Tris-(triméthylsilyl)amine |
| Otité : | Quantité |
| Rdt : | Rendement |
| $TBAF.3H_2O$ : | Fluorure de tétrabutylammonium trihydraté |
| TBAT : | Difluorotriphénylsilicate de tétrabutylammonium |
| tBuOK : | tertiobutylate de potassium |
| THF : | Tétrahydrofurane |

Au cours de cette étude, différents modes d'addition des réactifs ont été utilisés. Dans un souci de simplification, ces différentes méthodes ont été identifiées comme suit:

Procédure A

Dans le cas des bases usuelles (Par exemple amidures de type $LiN(SiMe_3)_2$ ou de tBuOK,) la base (en général 1M dans THF) est additionnée sur un mélange fluoroforme / électrophile / Solvant.
Dans le cas du système $N(SiMe_3)_3$ / $M^+ F^-$, l'amine silylée, en solution dans le THF, est coulée sur le mélange fluorure / électrophile / Solvant.

Procédure C

Cette méthode d'addition a surtout été utilisée pour l'action du système de type $N(SiMe_3)_3$ / $F^-$ sur des substrats baso-sensibles (cf. cidessus) tels que les dérivés benzyliques ou les cétones énolisables. La solution amine silylée / électrophile / solvant (En général, THF) est alors additionnée sur le mélange fluorure / fluoroforme / Solvant.

**Généralités**

Tous les réactions décrites ont été effectuées sous azote.
Tous les solvants et réactifs sont utilisés anhydres:

- ➪ le diméthylformamide est distillé sur $CaH_2$ puis conservé sur tamis moléculaire 4Å et sous azote.
- ➪ le tétrahydrofurane est distillé sur Na / benzophénone puis conservé sur tamis moléculaire 4Å et sous azote.

Les différents composants des systèmes basiques utilisés sont commerciaux:

- $LiN(SiMe_3)_2$ (1M dans THF), 100ml, ALDRICH.
- tBuOK (1M dans THF), 50ml, ALDRICH.
- $N(SiMe_3)_3$ 98%,25g, ALDRICH.
- $Me_4NF$ anhydre, 10g, ACROS.

- TBAT 97%, 25g, ALDRICH.

Définition du système perfluoroalcoylant pour les dérivés chalcogénés

Dans le cas de tous les substrats soufrés ou séléniés, 2 types de conditions ont été appliquées :

Pour 1 mmol de substrat

- Rf en excès(de 1,4 mmol à 8,6 mmol soit / $HCF_3$ de 200 mg à 600 mg)$LiN(SiMe_3)_2$ (1M dans THF) (1,1 ml soit 1,1 mmol de base) / HMDZ (40 µl soit 0,2 mmol) / DMF (2ml). *Procédure A*
- (de 1,4 mmol à 8,6 mmol soit $HCF_3$ de 200 mg à 600 mg )amine persilylée (1,5 mmol soit $N(SiMe_3)_3$ 350 mg) dans 1 ml de THF/ base silicophile (1,5 mmol $Me_4NF$ soit 140 mg) / DMF (2 ml) ou THF (2 ml) ; *Procédure A.*

Les exemples non limitatifs suivants illustrent l'invention.

EXEMPLE N° 1 : Trifluorométhylation des substrats dichalcogénés

Procédure A

Le substrat modèle choisi pour les différents modes opératoires est le disulfure de di-*n*-octyle.

Plage de températures : -20°C à 30°C.

Concentration du substrat : 1 mmol dans 3 ml.

réaction type: $(C_8H_{17}S)_2$ / $N(SiMe_3)_3$ / $Me_4NF$

Dans un ballon monocol de 5ml maintenu sous azote, on charge le disulfure de di-*n*-octyle (291 mg, 1 mmol), le fluorure de tétraméthylammonium anhydre (140 mg, 1,5 mmol) puis 2ml de DMF anhydre. Le mélange réactionnèl est refroidi à 0°C puis le fluoroforme (200 mg, 2,9 mmol) est additionné bulle à bulle. Une solution de tris-(triméthylsilyl) amine (352 mg, 1,5 mmol) dans 1 ml de THF est ensuite injectée à 0°C. Le mélange réactionnel est agité pendant 5h30 à 0°C. Après remontée à température ambiante, on en effectue un dosage par R.M.N. $^{19}F$: le sulfure de trifluorométhyle et de *n*-octyle est obtenu avec un rendement brut de 76%.

*Traitement* : le mélange réactionnel brut est additionné d'eau (2 ml) et d'acide chlorhydrique 1N (0,5 ml). La phase aqueuse est extraite 3 fois à l'éther éthylique. Les phases organiques sont rassemblées, lavées une fois avec une solution aqueuse saturée de NaCl et 2 fois à l'eau, puis séchées sur $Na_2SO_4$. Après filtration, le solvant est évaporé sous pression réduite, à froid, pour fournir une huile brute (306 mg). Sa purification sur colonne de silice (éluant : éther de pétrole pur) permet d'isoler du disulfure de di-*n*-octyle (37mg), $n$-$C_8H_{17}SH$ (88 mg) et le sulfure de trifluorométhyle et de *n*-octyle (140 mg, 0,65 mmol, 65%).

les résultats sont rassemblés dans le tableau suivant :

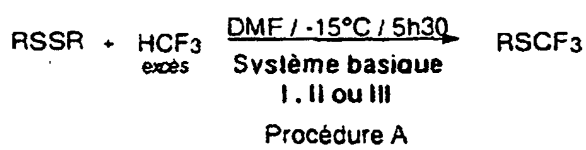


<u>Systèmes basiques :</u>

I : $LiN(SiMe_3)_2$ (1,1eq.) / HMDZ (0,2eq.)

II : $N(SiMe_3)_3$ (1,5eq.) / $Me_4NF$ (1,5eq.)

III : tBuOK (1,1eq.)

| substrat | produit désiré obtenu | Système basique | Rdt (%) [a] |
|---|---|---|---|
| 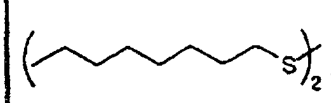 | 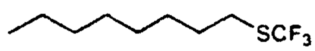 | I<br>II<br>II [b]<br>III | 51<br>73 (65)<br>66<br>54 (34) |
| 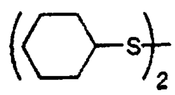 | 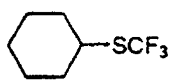 | I<br>II<br>III | 2 [c]<br>54<br>45 |
| 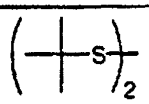 | 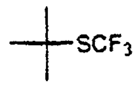 | II | 23 |
| 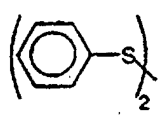 | 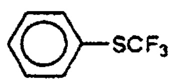 | I<br>II<br>III | 4 [e]<br>6<br>82 |
| Ph-Se-Se-Ph | Ph-Se-$CF_3$ | $N(SiMe_3)_3$ (1,5eq.) / $Me_4NF$ (1,5eq.) | 61 (47) |
| Ph-Se-Se-Ph | Ph-Se-$CF_3$ | tBuOK (1,1eq.) | 77 |

a) Rendement dosé par R.M.N. $^{19}F$ (Rendement isolé). b) Utilisation du THF comme solvant. c) Produit secondaire: $C_6H_{11}SCF_2H$: Rdt ($^{19}F$)=1%. d) Addition selon la procédure C. e) Produit secondaire: $PhSCF_2H$: Rdt ($^{19}F$)=23%.

EXEMPLE N° 2 : Trifluorométhylation des thiocyanates

La trifluorométhylation des thiocyanates et sélénocyanate est effectuée dans le diméthylformamide, pendant deux heures à basse température, en présence d'un excès de fluoroforme.

Conditions générales

$$R{-}SCN + \underset{excès}{HCF_3} \xrightarrow[\text{Système basique}]{\text{DMF / -10°C / 2h}} RSCF_3$$

Procédure A (en général)

Les deux systèmes basiques utilisés lors de ces essais, ont été le couple $N(SiMe_3)_3$ / $Me_4NF$, d'une part, et tBuOK, utilisé à titre de comparaison, d'autre part.

| substrat | Système basique | Rdt (%) [a] |
| --- | --- | --- |
| $CH_3(CH_2)_7$— | $N(SiMe_3)_3$ (1,5eq.) / $Me_4NF$ (1,5eq.) | 40 |
| | $N(SiMe_3)_3$ (1,5eq.) / $Me_4NF$ (1,5eq.) [b] | 11 |
| | $N(SiMe_3)_3$ (1,5eq.) / $Me_4NF$ (0,2eq.) | 5 |
| | tBuOK (1,1eq.) | 38 |
| 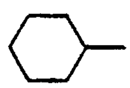 | $N(SiMe_3)_3$ (1,5eq.) / $Me_4NF$ (1,5eq.) | 25 |
| | tBuOK (1,1eq.) | 46 |
| 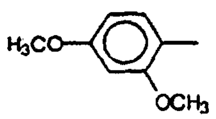 | $N(SiMe_3)_3$ (1,5eq.) / $Me_4NF$ (1,5eq.) | 23 (28) [b] |
| | tBuOK (1,1eq.) | 24 |

EXEMPLE N° 3 : Trifluorométhylation des sélénocyanates

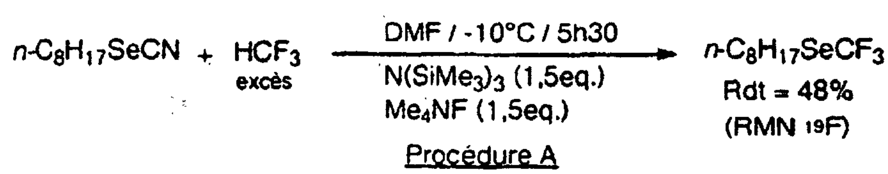


**TRIFLUOROMETHYLATION DE DERIVES CARBONYLES PAR $HCF_3$**

**Définition du système trifluorométhylant**

Dans le cas des dérivés carbonylés, le choix des conditions trrfluorométhylantes dépend de la baso-sensibilité du substrat.

**Substrats non baso-sensibles**

Pour 1 mmol de substrat

- $HCF_3$ (de 200 mg à 600 mg soit de 1,4 mmol à 8,6 mmol) / $LiN(SiMe_3)_2$ (1M dans THF) (1,1 ml soit 1,1 mmol de base) / HMDZ (40 µl soit 0,2 mmol) / DMF (2ml). *Procédure A.*
- $HCF_3$ (de 200 mg à 600 mg soit de 1,4 mmol. à 8,6 mmol) / $N(SiMe_3)_3$ (350 mg soit 1,5 mmol) dans 1 ml de THF/ F (de 0,2 à 1,5 mmol.). *Procédure A.*

Nature de F:

- $Me_4NF$ (de 19 mg à 140 mg) /DMF (2 mt).ou DMEU (2ml) ou DMPU (2 ml).
- $Me_4NF$ (idem) / DMF cata. (25 µl soit 0,3 mmol) / THF (2ml).
- CsF ( de 25 mg à 230 mg) / DMF (2 ml).
- TBAT (de 111 mg à 835 mg) I DMF (2 ml).
- TBAT (idem) / DMF cata. (25 µl soit 0,3 mmol) / THF (2 ml).
- TBAT (idem). / THF (2 ml).

**<u>Cétones énolisables</u>**

Dans ce cas, un seul système trifluorométhylant permet d'obtenir des alcools trifluorométhylés:

Pour 1 mmol de substrat

$HCF_3$ (de 200 mg à 600 mg soit de 1,4 mmol à 8,6 mmol) / $N(SiMe_3)_3$ (350 mg soit 1,5 mmol) dans 1 ml de THF/ $Me_4NF$ (140 mg soit 1,5 mmol) / DMF (2 ml). *Procédure C*

**<u>EXEMPLE N° 4 : Cétones non énolisables</u>**

Exemple utilisant $Me_4NF$ comme fluorure

Dans un ballon monocol de 5ml maintenu sous azote, on charge la benzophénone (183 mg, 1 mmol), le fluorure de tétraméthylammonium anhydre (22 mg, 0,24 mmol), 2ml de THF puis le diméthylformamide (25 µl, 0,3 mmol). Le mélange réactionnel est refroidi à -10°C puis le fluoroforme (400 mg, 5,7 mmol) est additionné bulle à bulle. Une solution de tris-(triméthylsilyl)amine (348 mg, 1,5 mmol) dans 1 ml de THF est ensuite injectée à -10°C. Le mélange réactionnel est agité pendant 1h00 à -10°C. Après remontée à température ambiante, on en effectue un dosage par R.M.N. $^{19}F$: le trifluorométhylcarbinol silylé et l'alcoolate trifluorométhylé sont obtenus avec des rendements bruts de 38% et 52%, respectivement.

*Traitement* : le mélange réactionnel brut est additionné d'eau (2 ml) et d'acide chlorhydrique 1N (0,5 ml). La phase aqueuse est extraite 3 fois à l'éther éthylique. Les phases organiques sont rassemblées, lavées une fois avec une solution aqueuse saturée de NaCl et 2 fois à l'eau, puis séchées sur $Na_2SO_4$. Après filtration, le solvant est évaporé sous pression réduite, à froid, pour fournir une huile brute (311 mg). La purification sur colonne de silice (éluant : éther de pétrole / acétone = 7 / 1) permet d'isoler la benzophénone (32 mg), le 1,1-diphényl-1-(triméthylsilyloxy)-2,2,2-trifluoroéthanol (194 mg, 0,6 mmol, 60%) et le 1,1-diphényl-2,2,2-trifluoroèthanol (18 mg, 0,07 mmol, 7%).b

: *PhCOPh / $LiN(SiMe_3)_2$ / HMDZ*

Dans un ballon monocol de 5ml maintenu sous azote, on charge la benzophénone (181 mg, 1 mmol) puis 2ml de DMF anhydre. Le mélange réactionnel est refroidi à -15°C puis le fluoroforme (300 mg, 4,3 mmol) est additionné bulle à bulle. L'hexaméthyldisilazane (50 µl, 0,24 mmol), puis $LiN(SiMe_3)_2$ (1,1 ml, 1,1 mmol) sont ensuite successivement injectés à -15°C. Le mélange réactionnel est agité pendant 1h00 à -15°C. Après remontée à température ambiante, on en effectue un dosage par R.M.N. $^{19}F$: le trifluorométhylcarbinol silylé et l'alcoolate trifluorométhylé sont formés avec des rendements bruts de 2% et 19%, respectivement.

Traitement est identique à celui ci-dessus.

Conditions générales (sauf exceptions signalées dans le tableau )

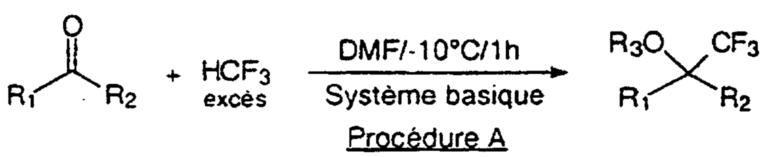

| $R_1$ | $R_2$ | Système basique | $R_3$ | Rendement [a] | observations |
|---|---|---|---|---|---|
| Ph | Ph | $LiN(SiMe_3)_2$ (1,1eq.) / HMDZ (0,2eq.) | H/$SiMe_3$ | 19/2 | |
| | | $N(SiMe_3)_3$ (1,5eq.) / $Me_4NF$ (1,5eq.) | H | 72 (50) | |
| | | $N(SiMe_3)_3$ (1,5eq.) / $Me_4NF$ (0,2eq.) | H/$SiMe_3$ | 57/28(47/33) | |
| | | $N(SiMe_3)_3$ (0,2eq.) / $Me_4NF$ (0,2eq.) | H | 6 | |
| | | $N(SiMe_3)_3$ (1,5eq.) / CsF (0,2eq.) | H/$SiMe_3$ | 37/66(18/68) | |
| | | $N(SiMe_3)_3$ (1,5eq.) / TBAT (0,2eq.) | H/$SiMe_3$ | 69/40(69/30) | |
| | | $N(SiMe_3)_3$ (1,5eq.) / TBAT (0,2eq.) | H | 71 | THF |
| | | $N(SiMe_3)_3$ (1,5eq.) / TBAT (0,2eq.) | H/$SiMe_3$ | 91/3(36/55) | solvant = THF + 0,3 eq. de DMF |
| | | $N(SiMe_3)_3$(1,5eq.) / TBAF.$3H_2O$(0,2eq.) | H | 5 | |
| | | tBuOK (1,1eq.) | H | 100 (76) | |
| | | tBuOK (1,1eq.) | H | 67 | dans le THF |
| 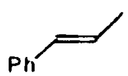 | Ph | $N(SiMe_3)_3$ (1,5eq.) / $Me_4NF$ (1,5eq.)<br>$(SiMe_3)_3$ (1,5eq.) / $Me_4NF$ (0,2eq.) | H<br>H/$SiMe_3$ | 30 (40)<br>68 (42/26) | |
| 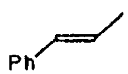 | 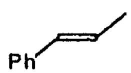 | $N(SiMe_3)_3$ (1,5eq.) / $Me_4NF$ (0,2eq.) | H/$SiMe_3$ | 40/22 (45) | d) Rdt en alcool silylé par $ClSiMe_3$. |
| 4-F-$C_6H_4$- | 4-F-$C_6H_4$- | $N(SiMe_3)_3$ (1,5eq.) / CsF (0,2eq.) | H/$SiMe_3$ | 43/44(0/64) | |
| 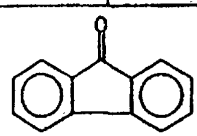 | | $N(SiMe_3)_3$ (1,5eq.) / CsF (0,2eq.) | $SiMe_3$ | 72 (57) | TT = 62 % |

a) Rendement dosé par R.M.N. 19F (et Rendement isolé).(%)

EXEMPLE N° 5 : Cétones non énolisables. Effet de solvant

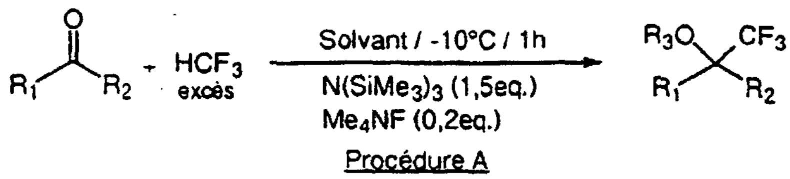


| $R_1$ | $R_2$ | Solvant | $R_3$ | Rendement (%) [a] |
|---|---|---|---|---|
| 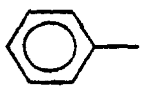 | 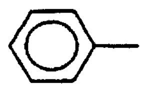 | DMF | H/$SiMe_3$ | 80/10 |
| | | THF + DMF (0,3eq.) | H/$SiMe_3$ | 52/38 (7/60) |
| | | DMEU | H/$SiMe_3$ | 41/34 |
| | | DMPU | H/$SiMe_3$ | 51/16 |
| 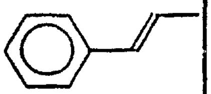 | 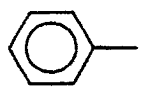 | DMF | H/$SiMe_3$ | 68/0 (42/26) |
| | | THF + DMF (0,3eq.) | H/$SiMe_3$ | 17/2 |
| 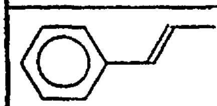 | 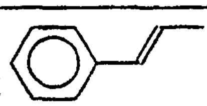 | DMF | H/$SiMe_3$- | 40/22 (45) [b] |
| 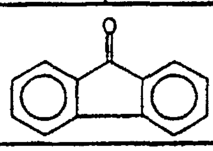 | | THF + DMF (0,3eq.) | $SiMe_3$ | 83 (75) [d] |

a) Rendement dosé par R.M.N. $^{19}F$ (Rendement isolé). b) Rendement en alcool silylé après traitement par $ClSiMe_3$ (1eq.). d) Récupération de 19% de fluorénone.

EXEMPLE N° 6 : Cétones non énolisables cas des quinols

Les conditions opératoires utilisées sont celles appliquées dans les cas des cétones, l'hydrolyse finale s'effectuant en milieu neutre ou acide.

Traitement post-réactionnel en milieu neutre :

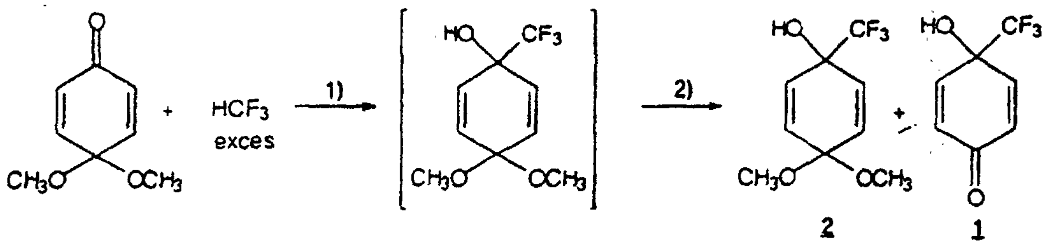


2 (45%) 1 (22%)

1) $N(SiMe_3)_3$ (1,5eq.)/.$Me_4NF$ (0,2eq.) dans le DMF à -10°C pendant 1 heure (ProcédureA). 2) $H_2O$.
entre parenthèses: rendement en produit isolé.

Après traitement aqueux en milieu neutre et chromatographie sur silice, on récupère l'alcool trifluorométhylé 2 (45%) et la cétone 1 issue de l'hydrolyse de la fonction cétal de 2 (22%).

Traitement post-réactionnel en milieu acide :

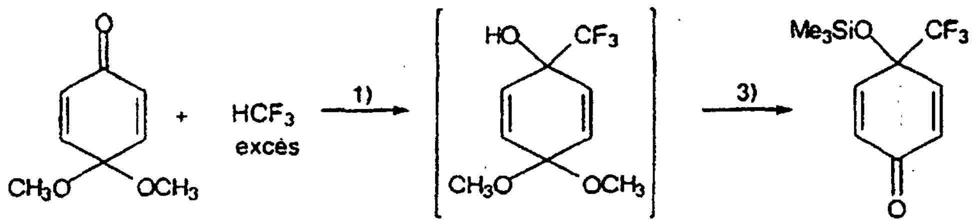


3 (44%)

1) $N(SiMe_3)_3$ (1,5eq.)/.$Me_4NF$ (0,2eq.) dans le DMF à -10°C pendant 1 heure (Procédure A). 3) HCl 1N
entre parenthèses: rendement en produit isolé.

Si le traitement s'effectue en milieu acide (HCl 1N), la réaction de trifluorométhylation étant menée dans des conditions identiques, la cétone 3 est obtenue avec un rendement de 44% après chromatographie sur silice.

<u>Trifluorométhylation de quinols de type 4-méthoxy-4-méthyl cyclohexa-2,5-dién-1-one</u>

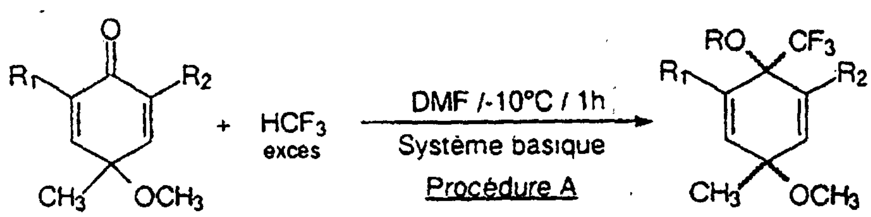

| $R_1$ | $R_2$ | Système basique | R | Rendement (%) [a] | R.M.N. $^{19}F$ [b] |
|---|---|---|---|---|---|
| H | H | $N(SiMe_3)$ (1,5eq.) / $Me_4NF$ (0,2eq.) | $SiMe_3$ | 64 (44)[c] d.e.= 0% } 26 / 38 | -81,23<br>-81,90 |
| | | tBuOK (1,1eq.) | H | 26 (42)<br>33 (33) | -80,64<br>-81,27 |
| tBu | H | $N(SiMe_3)$ (1,5eq.) / TBAT (0,2eq.) | $SiMe_3$ | 52 (32)[c] d.e.= 60% } 8 / 44 | -72,58<br>-75,24 |

a) Rendements des 2 stéréoisomères dosés par R.M.N. $^{19}F$ (Rendement isolé). b) dans $CDCl_3$, ppm (ref.: $CFCl_3$). c) Rendement isolé correspondant au mélange des deux stéréoisomères.

EXEMPLE N° 7: Cétones énolisables :

Conditions générales (sauf exceptions signalées dans le tableau n°8)

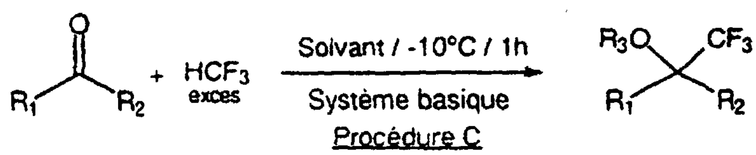


Procédure C :

Le substrat modèle choisi pour les différents modes opératoires est l'acétophénone.

Plage de températures : -20°C à 30°C.

Concentration du substrat : 1 mmol dans 3 ml.

PhCOMe / $N(SiMe_3)_3$ / $Me_4NF$

Dans un ballon monocol de 5ml maintenu sous azote, on charge le fluorure de tétraméthylammonium anhydre (140 mg, 1,5 mmol) et 2 ml de DMF anhydre. Le mélange est refroidi à -10°C puis le fluoroforme (400 mg, 4,3 mmol) est additionné bulle à bulle. Une solution de tris-(triméthylsilyl)amine (350 mg, 1,5 mmol) et d'acétophénone (120 mg, 1 mmol) dans 1 ml de THF est ensuite injectée à -10°C. Le mélange réactionnel est agité à -10°C pendant une heure. Après remontée à température ambiante, on en effectue un dosage par R.M.N. $^{19}F$: l'alcoolate trifluorométhylé est formé avec un rendement brut de 26%.

| $R_1$ | $R_2$ | Système Basique | $R_3$ | Rdt (%) [a] |
|---|---|---|---|---|
| Ph | $CH_3$- | $N(SiMe_3)_3$ (1,5eq.) / $Me_4NF$ (1,5eq.) | H | 26 |
| Ph | $CH_3CH_2$- | $N(SiMe_3)_3$ (1,5eq.) / $Me_4NF$ (1,5eq.) | H | 28 |
| Ph | 2-$CH_3$-$C_6H_5$ | $N(SiMe_3)_3$ (1,5eq.) / $Me_4NF$ (1,5eq.) | H | 11 |
| 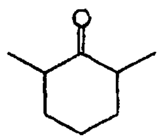 | | $N(SiMe_3)_3$ (1,5eq.) / $Me_4NF$ (1,5eq.)<br>$N(SiMe_3)_3$ (1,5eq.) / $Me_4NF$ (0,2eq.) | H/$SiMe_3$<br>H/$SiMe_3$ | 36/14<br>3/42 [c] |

a: Rendement dosé par R.M.N. $^{19}F$ (Rendement isolé) c: Après 10 jours à température ambiante.

Dans le cas des cétone énolisable la procédure C et l'utilisation de $Me_4NF$ donne de meilleur résultats. le caractère aromatique de la cétone n'est pas favorable à l'utilisation sous-stoechiométrique des anions silicophiles et notamment du fluorure.

EXEMPLE N° 8 : Trifluorométhylation des N-formyl amides (DMF)

Procédure A

Plage de températures : -20°C à 30°C.

Concentration du substrat : 1 mmol dans 3 ml.

DMF / $N(SiMe_3)_3$ / TBAT

Dans un ballon monocol de 5ml maintenu sous azote, on charge le difluorotriphénylsilicate de tétrabutylammonium (111 mg, 0,2 mmol) et 2ml de diméthylformamide. Le mélange réactionnel est refroidi à -10°C puis le fluoroforme (400 mg, 5,7 mmol) est additionné bulle à bulle. Une solution de tris-(triméthylsilyl)amine (348 mg, 1,5 mmol) dans 1 ml de THF est ensuite injectée à - 10°C. Le mélange réactionnel est agité pendant 1h00 à -10°C. Après remontée à température ambiante et addition de chlorotriméthylsilane (130 µl, 1 mmol), on en effectue un dosage par R.M.N. $^{19}F$: l'intermédiaire tétrahédrique silylé est obtenu avec un rendement brut de 27%.

EXEMPLE N° 9 : Trifluorométhylation des N-formyl amides N-méthyl morpholine / $N(SiMe_3)_3$ / TBAT

Dans un ballon monocol de 5ml maintenu sous azote, on charge la N-méthyl morpholine (117 mg, 1 mmol), le difluorotriphénylsilicate de tétrabutylammonium (110 mg, 0,2 mmol) et 2ml de THF. Le mélange réactionnel est refroidi à -10°C puis le fluoroforme (400 mg, 5,7 mmol) est additionné bulle à bulle. Une solution de tris-(triméthylsilyl) amine (351 mg, 1,5 mmol) dans 1 ml de THF est ensuite injectée à -10°C. Le mélange réactionnel est agité pendant 1h00 à -10°C. Après remontée à température ambiante, on en effectue un dosage par R.M.N. $^{19}F$: le trifluorométhylcarbinol silylé et l'alcoolate trifluorométhylé sont obtenus avec des rendements bruts de 49% et 15%, respectivement. On injecte alors du chlorotriméthylsilane (130 µl, 1 mmol) et on laisse sous agitation pendant une heure à température ambiante.

Traitement : le mélange réactionnel brut est additionné d'eau (2 ml). La phase aqueuse est extraite 3 fois à l'éther éthylique. Les phases organiques sont rassemblées, lavées une fois avec une solution aqueuse saturée de

NaCl et 2 fois à l'eau, puis séchées sur $Na_2SO_4$. Après filtration, le solvant est évaporé sous pression réduite, à froid, pour fournir une huile brute (534 mg). La purification sur colonne de silice (éluant : éther de pétrole / acétone = 9 / 1) permet d'isoler l'intermédiaire tétrahédrique trifluorométhylé silylé avec un rendement de 60%.

EXEMPLE N° 10 : Trifluorométhylation des phtalimides

Procédure A

Le substrat est le N-méthyl phtalimide.
Plage de températures : -20°C à 30°C.
Concentration du substrat : 1 mmol dans 3 ml.

N-méthyl phtalimide / $N(SiMe_3)_3$ / $Me_4NF$

Dans un ballon monocol de 5ml maintenu sous azote, on charge le N-méthyl phtalimide (160 mg, 1 mmol), le fluorure de tétraméthylammonium anhydre (22 mg, 0,24 mmol), 2ml de DMF. Le mélange réactionnel est refroidi à - 10°C puis le fluoroforme (200 mg, 2,9 mmol) est additionné bulle à bulle. Une solution de tris-(triméthylsilyl)amine (351 mg, 1,5 mmol) dans 1 ml de THF est ensuite injectée à -10°C. Le mélange réactionnel est agité pendant 1h00 à -10°C. Après remontée à température ambiante, on en effectue un dosage par R.M.N. $^{19}F$: l'alcool trifluorométhylé silylé est obtenu avec un rendement brut de 7%.
Traitement : le mélange réactionnel brut est additionné d'eau (2 ml) et d'acide chlorhydrique 1N (0,5 ml). La phase aqueuse est extraite 3 fois à l'éther éthylique. Les phases organiques sont rassemblées, lavées une fois avec une solution aqueuse saturée de NaCl et 2 fois à l'eau, puis séchées sur $Na_2SO_4$. Après filtration, le solvant est évaporé sous pression réduite, à froid, pour fournir une huile brute (442 mg). La purification sur colonne de silice (éluant : éther de pétrole / éther éthylique = 1/1) permet d'isoler le N-méthyl phtalimide (130 mg) et l'alcool trifluorométhylé silylé (37 mg, 0,14 mmol).

EXEMPLE N° 11 : Utilisation des intermédiaires tétrahédriques

Le substrat modèle choisi pour les différents modes opératoires est la benzophénone.
Plage de températures : -20°C à 30°C.
Concentration du substrat : 1 mmol dans 3,5 ml.

EXEMPLE N° 12 : DMF / $N(SiMe_3)_3$ / TBAT / PhCOPh

Dans un ballon monocol de 5ml maintenu sous azote, on charge le triphényldifluorosilicate de tétrabutylammonium (111mg, 0,2 mmol) et 2 ml de diméthylformamide. Le mélange réactionnel est refroidi à -10°C puis le fluoroforme (400 mg, 5,7 mmol) est additionné bulle à bulle. Une solution de tris-(triméthylsilyl)amine (348 mg, 1,5 mmol) dans 1 ml de THF est ensuite injectée à - 10°C. Le mélange réactionnel est agité pendant 1h00 à -10°C. Après remontée à température ambiante, on en effectue un dosage par R.M.N. $^{19}F$: l'intermédiaire tétrahédique trifluorométhylé sous forme anionique et silylée est obtenu avec des rendements bruts respectifs de 18% et 87% (rendements calculés par rapport à la benzophénone). On injecte alors une solution de benzophénone (182 mg, 1 mmol) dans 0,5 ml de DMF. Le mélange réactionnel est agité à température ambiante pendant 24h00 puis dosé par R.M.N. $^{19}F$: le trifluorométhylcarbinol silylé et l'alcoolate trifluorométhylé sont obtenus avec des rendements bruts de 21% et 71 %, respectivement. Après traitement et purification classiques (cf. exemple n°17), on isole le 1,1-diphényl-1-(triméthylsilyloxy)-2,2,2-trifluoroéthanol (32 mg, 0,32 mmol, 10%) et le 1,1-diphényl-2,2,2-trifluoroéthanol (219 mg, 0,87 mmol, 87%).

: N-méthyl morpholine / $N(SiMe_3)_3$ / TBAT / PhCOPh

Dans un ballon monocol de 5ml maintenu sous azote, on charge le triphényldifluorosilicate de tétrabutylammonium (111mg, 0,2 mmol), la N-méthyl morpholine (117 mg, 1 mmol) et 2 ml de THF. Le mélange réactionnel est refroidi à -10°C puis le fluoroforme (300 mg, 4,3 mmol) est additionné bulle à bulle. Une solution de tris-(triméthylsilyl) amine (352 mg, 1,5 mmol) dans 1 ml de THF est ensuite injectée à -10°C. Le mélange réactionnel est agité pendant 1h00 à -10°C. Après remontée à température ambiante, on en effectue un dosage par R.M.N. $^{19}F$: l'intermédiaire tétrahédique trifluorométhylé sous sa forme silylée est obtenu avec un rendement brut de 37% (rendement calculé par rapport à la benzophénone). On injecte alors une solution de benzophénone (180 mg, 1 mmol) dans 0,5 ml de THF. Le mélange réactionnel est agité à température ambiante pendant 24h00 puis dosé par R.M.N. $^{19}F$: l'alcoolate trifluorométhylé est obtenu avec un rendement brut de 20% mais il reste 0,23mmol d'intermédiaire tétraédrique résiduel.

EXEMPLE N° 13 Déprotonation du fluoroforme par $KN(SiMe_3)_2$ et silylation de l'intermédiaire tétraédrique par un agent silylant:

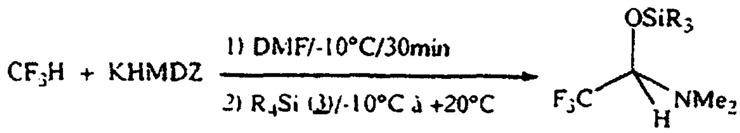


(1) (2) (4)

| $R_4Si$ | (1)/(2)/(3) (b) | RR(4) % (a) |
|---|---|---|
| $Me_3SiCl$ | 4/1/1.1 | 48.5 |
| $tBuMe_2SiCl$ | 4/1/1.1 | -55 |
| $(Me_3Si)_3N$ | 4/1/1.1 | 33.5 |

(a) dosage avec étalon interne en RMN du fluor
(b) rapport molaire

***REACTIFS***

| | |
|---|---|
| Fluoroforme | 28 mmoles (2g) |
| KHMDZ | 7 mmoles |
| Agent silylant (b) | 7.7 mmoles |
| DMF anhydre | 30 ml |
| (a) Agents silylants testés: $Me_3SiCl$, $(Me_3Si)_3N$, $Me_2{}^tBuSiCl$ | |

***MODE OPERA TOIRE***

7 mmoles de base sont chargées dans le réacteur parfaitement agité de 100 ml; 30 ml de DMF anhydre y sont ajoutés via une seringue. Le milieu réactionnel est ensuite porté à -10 °C et 4 équivalents de fluoroforme sont alors additionnés par bullage, sur une période de 20 minutes.

Le milieu réactionnel est laissé sous agitation à -10 °C pendant 30 minutes.

L'agent silylant est ensuite additionné goutte à goutte via une seringue. On laisse alors le milieu réactionnel revenir à température ambiante, puis on dose la quantité d'hémiaminal formé (RMN $^{19}F$ avec comme étalon interne $PhOCF_3$).

***ISOLEMENT DE*** $CF_3CH(OSiMe_2{}^tBu)NMe_2$

Le milieu réactionnel est coulé dans 30 ml d'eau déminéralisée glacée puis extrait 3 fois avec 30 ml d'acétate d'éthyle. Les phases organiques sont lavées à l'eau déminéralisée glacée jusqu'à totale disparition du DMF (contrôle CPG), puis, séchées sur $MgSO_4$ et concentrées à l'évaporateur rotatif (θ°amb, 185 mbar). Le produit est isolé pur par distillation et a été caractérisé par RMN $^1H$, $^{13}C$ et $^{19}F$.

L'utilisation de base en ***quantité catalytique*** a ensuite été expérimentée selon un mode opératoire identique au précédent:

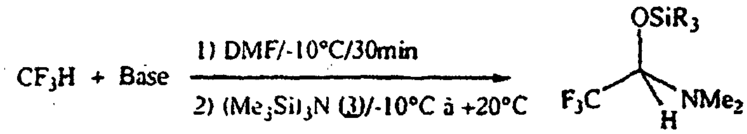


(1) (2) (4)

↻ Résultats:

| Base | (1)/(2)/(3) (b) | RR(4) % (a) | Commentaires |
|---|---|---|---|
| KHMDZ | 4/1/1,1 | 33.5 | base en quantité stoechiométrique |
| KHMDZ | 4/0,1/1,1 | 59 | base en quantité catalytique |

(a) dosage avec étalon interne en RMN du fluor

(b) rapport molaire

EXEMPLE N° 14 Addition de l'anion CF3 issu de $CF_3SiR_3$ sur le DMF :

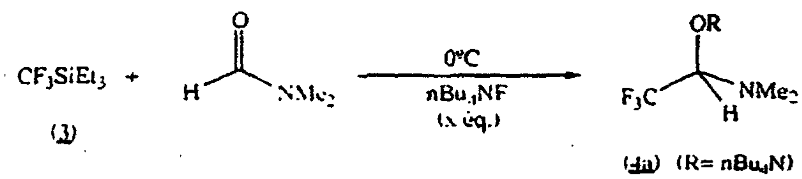


(3)

(4a) (R= $nBu_4N$)
(4b) (R= $SiEt_3$)

| x. (éq.) | Analyse RMN $^{19}F$ (25°C) (1) |
|---|---|
| 1 | $CF_3H$ (RR = 80%), $CF_3SiEt_3$ (TT = 100%) |
| 0,1 | $CF_3H$ (RR = 22%), $CF_3SiEt_3$ (TT = 100%), et 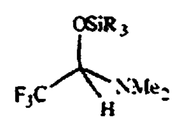 RR= 60% |

(1) dosage RMN $^{19}F$ avec étalonnage interne.

***MODE OPERATOIRE***

On introduit dans un réacteur de 25 ml sous atmosphère d'argon, 15 ml de DMF anhydre et 0,38 ml de $CF_3SiEt_3$ ( 2 mmol). Le milieu réactionnel est refroidi à 0°C et on ajoute goutte à goutte 0,2 ml d'une solution de nBu4NF 1M dans le THF (0,2 mmol).

Le milieu réactionnel est laissé sous agitation à 0°C durant 30 min et on laisse remonter ensuite à température ambiante.

La quantité de $CF_3CH(OSiEt_3)NMe_2$ est déterminé par dosage en RMN 19F en présence d'un étalon interne ($\delta$ = 2,9 ppm /TFA, Rdt = 60%).

Cet intermédiaire a été isolé et caractérisé d'une façon identique à celle décrite dans le cas de de $CF_3H$

(OSiMe$_2$$^t$Bu)NMe$_2$.

**EXEMPLE N° 15 retour à l'anion intermédiaire tétraèdrique à paritir du dérivé silylés**

Dans un réacteur de 5 ml sous atmosphère d'argon, on introduit $CF_3CH(OSiMe_2{}^tBu)NMe_2$ (0,135 g) en solution dans 3 ml de DMF anhydre. Le milieu réactionnel est refroidi à -10°C et on additionne 50 mg de tBuONa. Le milieu réactionnel est analysé par RMN 19F basse température et on note l'apparition de $CF_3CH(O^-)NMe_2$ ( $\delta$ = 1 ppm /TFA).

**EXEMPLE N° 16 obtention de l'anion tétraèdrique à partir de l'hémiaminal**

**Preparation de l'hemiaminal à partir de fluoral anhydre**

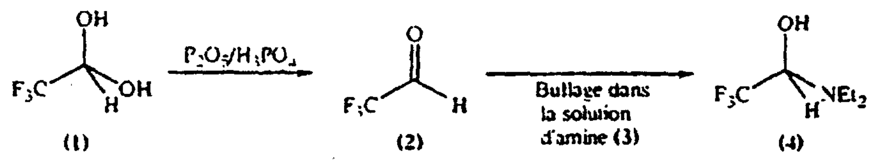


| | |
|---|---|
| $P_2O_5$ | 30 g |
| $H_3PO_4$ | 30g |
| hydrate de fluoral 75 % p/p | 6 g soit 38.8 mmol |
| $Et_2NH$ | 1,085g soit 44,9 mmol |
| THF anhydre | 12 ml |

30 g de $P_2O_5$ et 30g de $H_3PO_4$ sont chargés dans un tricol de 250 ml sous atmosphère inerte d'argon; ce mélange est porté à 95 °C (θ° du bain d'huile). Le montage est relié à un tricol de 50 ml surmonté d'un condenseur à carboglace par un tuyau téflon.

La diéthylamine et, éventuellement le THF, sont chargés dans un tricol de 50 ml; ce milieu réactionnel est porté à -40 °C.

Lorsque ces deux températures sont atteintes, l'hydrate de fluoral est additionné goutte à goutte au mélange déshydratant (ampoule de coulée); le bullage commence alors dans la solution d'amine. Cette addition terminée, on laisse le tout revenir à température ambiante. L'hémiaminal formé est alors dosé en RMN $^{19}$F.

$$RR(CF_3CH(OH)NEt_2) = 76\%$$

**TRIFLUOROMETHYLATION**

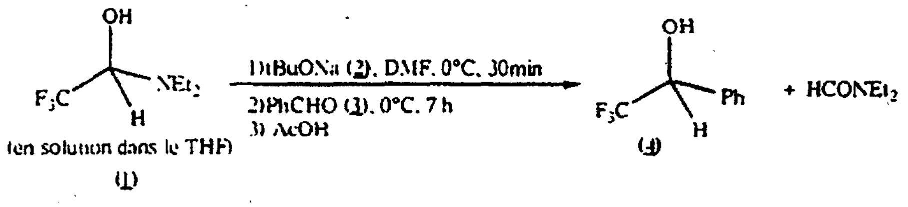

| | | |
|---|---|---|
| tBuONa | - 4,5 mmol | 0.43 g |
| solution d'hémiaminal dans le THF | - 3,7 mmol | 4,3 g |
| DMF anhydre | - | 25 ml |
| benzaldéhyde | - 3,5 mmoles | 0,37 g |
| acide acétique 100% | - 6 mmoles | 0,36 q |

4,5 mmoles de tBuONa sont chargées dans un réacteur agité de 100 ml et, 25 ml de DMF anhydre y sont ajoutés.

Le milieu réactionnel est mis sous agitation et porté à 0 °C.

La solution d'hémiaminal est alors ajoutée goutte à goutte, via une seringue, au travers d'un septum. Le milieu réactionnel est maintenu à 0 °C pendant 30 minutes.

3,5 mmoles de benzaldéhyde sont alors additionnées lentement, goutte à goutte, via une seringue (légère exothermie). Le milieu réactionnel est maintenu à 0 °C, et la réaction est suivie en CPG,

Au bout de 7 heures de réaction, 6,0 mmoles d'acide acétique sont additionnés lentement au milieu réactionnel qui est alors laissé à température ambiante et dosé en CPG.

$$RR(CF_3CH(OH)Ph) = 48\%$$

$$TT\ (PhCHO) = 84\ \%$$

$$RT\ (CF_3CH(OH)Ph) = 57\ \%$$

**EXEMPLE N° 17 : ROLE DE LA NATURE DU SOLVANT**

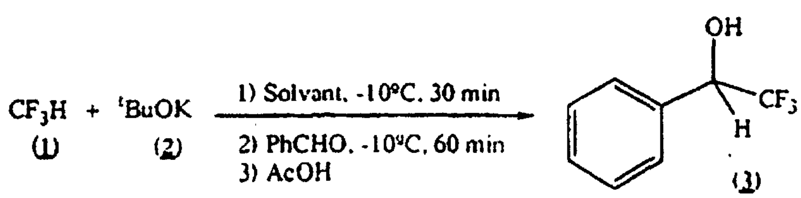


A une solution convenablement agitée (400t/min) de tBuOK (0,53g, 4,7 mmol) dans 30 ml de solvant anhydre(S) est additionnée, à -10°C, du fluoroforme (3g, 42,85 mmol). Le milieu réactionnel est laissé sous agitation 30 minutes à -10°C, avant d'additionner le benzaldéhyde (0,47g, 4,4 mmol).
La solution est laissée sous agitation à -10°C, 60 minutes supplémentaires, avant addition d'acide acétique (0,5 ml).
La composition du mélange est déterminée par dosage CGL avec étalonnage interne:

| Solvant | RR (3) % |
|---|---|
| THF | 25 |
| DMF | 57 |
| N-Formylpipéridine | 5 |

**EXEMPLE N° 18 : ROLE DE LA NATURE DE LA BASE**

⇨ ***Cation associé*** (Mode opératoire général)

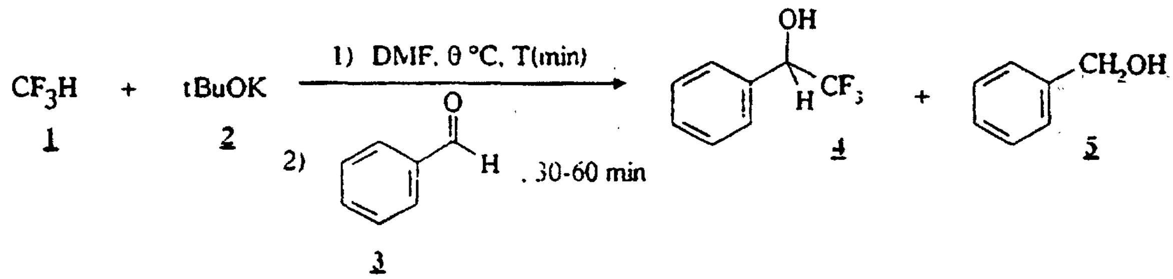

1 2 3 4 5

| tBuOM *(a)* | θ °C | T (min) | TT (3) (%) | RR (4) (%) | RT (4) (%) | RR (5) (%) | RT (5) (%) |
|---|---|---|---|---|---|---|---|
| tBuOK | -20 | 30 | 88 | 64 | 73 | traces | - |
| tBuONa | -20 | 30 | 83 | 59 | 71 | traces | - |
| tBuOLi | -20 | 30 | 32.5 | 13 | 40 | traces | - |

***(a)*** CF.$_3$H/tBuOM/PhCOH (9/1,1/1).

**EXEMPLE N° 19: MISE EN EVIDENCE ET ROLE DE L'INTERMEDIAIRE TETRAEDRIQUE**

➊ ***Synthèse d'hémiaminal du fluoral et de dérivés***

A une solution convenablement agitée de base dans 30 ml de DMF anhydre, on additionne à -10°C du fluoroforme (3g, 42,85 mmol). Cette solution est maintenue à -10°C durant 30 min puis on additionne goutte à goutte à cette même température:

- AcOH (0,37g, 6,2 mmol) dans le cas où R= H (base: KH/DMSO, 5,7 mmol),
- $Me_3SiCl$ (1,3 ml, 10,25 mmol) dans le cas où R= $Me_3Si$ (base: KHMDZ, 7 mmol)
- $SO_2$ (0,8g, 12,5 mmol) dans le cas où R= $SO_2^-K^+$ (base: KH/DMSO, 5,9 mmol).

Le milieu réactionnel est alors maintenu à cette même température durant 30 minutes avant de le laisser remonter à température ambiante.
Les produits formés ont été identifiés par RMN $^1H$, $^{19}F$, $^{13}C$.

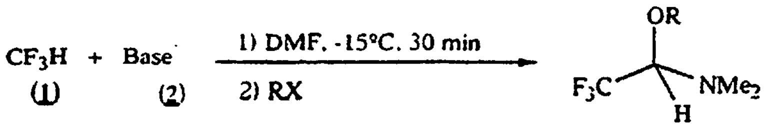


RX= AcOH, (3a), R= H
RX= $Me_3SiCl$, (3b). R= $Me_3Si$
RX= $SO_2$. (3c). R= $SO_2^-$ $K^+$

| RX | RR(dosé) |
|---|---|
| AcOH | 3a, 76% |

(suite)

| RX | RR(dosé) |
|---|---|
| $Me_3SiCl$ | 3b, 79% |
| $SO_2$ | 3c, 77% |

❷ ***Synthèse d'hydrate de fluoral***

A une solution convenablement agitée de tBuOK (5 mmol) dans un solvant anhydre (30 ml), maintenue à -15°C, on ajoute du fluoroforme (3g, 42,85 mmol). Après 30 min à cette température, le milieu réactionnel est acidifié par 2 ml d'acide sulfurique. Le tableau suivant donne les résultats en hydrate de fluoral en fonction des Daramètres opératoires:

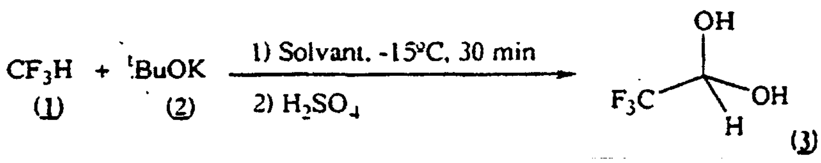


| Solvant | RR(3)(1)% |
|---|---|
| DMF | 60 |
| 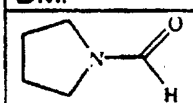 | 56 |
| 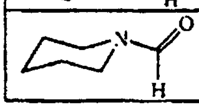 | 52 |

(1) Dosage RMN $^{19}F$ avec étalon interne.

**EXEMPLE N° 20 :** ***Synthèse de 2,2,2-trifluoroacetophénone***

A une solution convenablement agitée (400t/min) de KHMDZ (1,15g, 5,75 mmol) dans 30 ml de DMF anhydre est additionnée à -10°C, du fluoroforme (3,0g, 43 mmol). Le milieu réactionnel est laissé sous agitation 30 minutes à -10°C, avant d'additionner goutte à goutte le benzoate de méthyle (0,51g, 3,75 mmol).
La solution est laissée sous agitation à -10°C, 1,5 heures supplémentaires, avant addition d'acide acétique (0,6 ml).
Après un traitement classique du milieu réactionnel (extraction et distillation), la trifluoroacétophénone est isolée avec un rendement de 55%.

**EXEMPLE N° 21 :** ***1,1,1,3,3,3-Hexafluoro-2-phényl-2-propanol***

A une solution convenablement agitée (400t/min) de dimsylate de potassium (5,85 mmol) dans 30 ml d'un mélange DMF/DMSO anhydre (2/1), est additionnée à -10°C, du fluoroforme (3,0g, 43 mmol). Le milieu réactionnel est laissé sous agitation 30 minutes à -10°C, avant d'additionner goutte à goutte la trifluoroacétophénone (0,615g, 3,5 mmol).
La solution est laissée sous agitation à -10°C, 1h10 supplémentaire, avant addition d'acide acétique (0,6 ml).
La composition du mélange est déterminée par dosage RMN 19F et CGL avec étalonnage interne:

$$TT(PhCOCF_3) = 35\%$$

$$RR(PhCOH(CF_{.3})_2) = 79\%$$

$$RT(PhCOH(CF._3)_2) = 44\%$$

**EXEMPLE N° 22 : AUTRES HALOFORMES**

**Mode opératoire générale**

Dans un réacteur parfaitement agité de 500 ml, comportant une agitation mécanique (650 t/min) et maintenu sous balayage d'azote, on introduit environ 5 g de tertiobutylate de potassium, puis 120 ml de DMF anhydre. Le milieu réactionnel est alors refroidi à -40°C au moyen d'un bain acétone-carboglace. Environ 5 g de benzaldéhyde sont alors introduits goutte à goutte, puis 3 à 4 équivalents d'haloforme, par bullage au sein du milieu réactionnel s'il est gazeux ($CCl_2FH$, $CF._3CF._2H$), au goutte à goutte s'il est liquide ($CCl_3H$). Après une heure d'agitation entre -40 et -45°C, on ajoute goutte à goutte 5 ml d'acide acétique concentré puis on laisse revenir à température ambiante. Le milieu réactionnel brut est analysé en CGL, puis par couplage CGL/SM afin d'identifier le produit et les sous-produits formés.
On dilue le milieu réactionnel dans 150 ml d'eau, puis on extrait les produits avec de l'acétate d'éthyle (3x170 ml). Les phases organiques rassemblées sont alors lavées 4 à 6 fois avec 100 ml d'eau afin d'éliminer le DMF (contrôle CGL), puis deux fois avec 50 ml de solution saturée en NaCl. La phase organique est alors séchée sur $MgSO_4$ anhydre durant 30 à 60 minutes, puis filtrée sur verre fritté.
Si la température d'ébullition du composé synthétisé est suffisamment élevée, l'acétate d'éthyle peut être évaporé à l'évaporateur rotatif, sous un vide de 20 mm Hg et à une température de 35°C ; dans le cas contraire, l'acétate d'éthyle est alors distillé à pression atmosphérique.
On effectue une distillation fractionnée sous un vide d'environ 15 mm Hg. On isole ainsi le carbinol avec une pureté supérieure à 90%.

**Résultats**

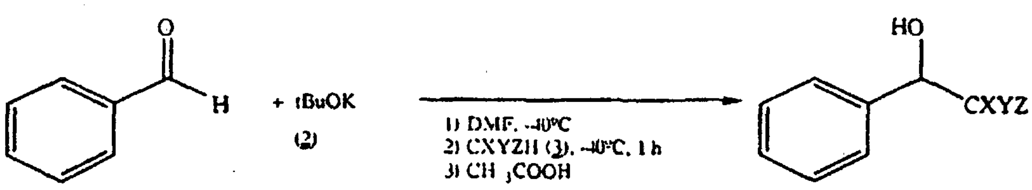


(1) (4)

| CXYZH | point d'ébullition(b) | (1)(a) | (2)(a) | (3)(a) | TT (1) (%) | RR (4) (%) |
|---|---|---|---|---|---|---|
| $CF._3H$ | -80°C | 1 | 1 | 8,6 | 94 | 67 |
| $CF._3CF._2H$ | -50°C | 1 | 1 | 4 | 98,5 | 71 |
| $CCl_2FH$ | 10°C | 1 | 1 | 3 | ~100 | 64 |
| $CCl_3H$ | 60°C | 1 | 1 | 3,6 | ~100 | 62 |

(a)-Nombre d'équivalents.
(B)- Arrondi et sous pression atmosphérique

**EXEMPLE N° 23 :** addition de l'anion -CF3 (ex $CF_3SiMe_3$) sur le DMF :

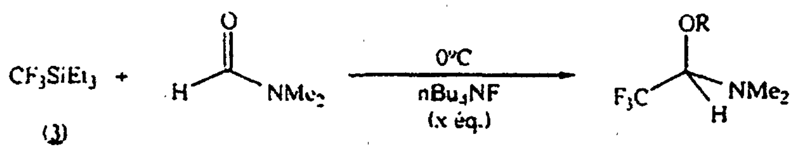


(3)

(4a) (R= nBu4N)
(4b) (R= SiEt3)

| Essai | x. (éq.) | Analyse RMN $^{19}F$ (25°C) (1) |
|---|---|---|
| 95RON57A | 1 | $CF_3H$ (RR = 80%), $CF_3SiEt_3$ (TT = 100%) |
| 95RON57B | 0,1 | $CF_3H$ (RR = 22%), $CF_3SiEt_3$ (TT = 100%), et 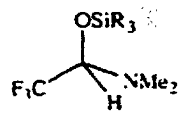 RR= 60% |

(1) dosage RMN $^{19}F$ avec étalonnage interne.

**EXEMPLE N° 24 : ESSAIS DE TRIFLUOROMETHYLATION UTILISANT DES ALCOOLATES COMME BASE SILICOPHILE**

**Procédure A**

Dans le cas du système $N(SiMe_3)_3$ / B $M^+$ (acétate et carbonates), l'amine silylée, en solution dans le THF, est coulée sur un mélange B $M^+$ / électrophile / fluoroforme / solvant.

**Procédure B**

Dans le cas du système $N(SiMe_3)_3$ / RONa, le fluoroforme est additionné sur un mélange $(N(SiMe_3)_3$ / RONa électrophile / solvant.

Dans le cas de l'utilisation comparative des alcoolates (RONa) seuls comme base le fluoroforme es additionné sur un mélange RONa / électrophile / solvant.

**RESULTATS**

| Essai | BASE | Rdt (%) | OH/OSiMe3 |
|---|---|---|---|
| 1 | MeOna (1,5eq.) | 29 | 100/0 |
| 2 | EtONa (1,5eq.) | 23 | 100/0 |
| 3 | iPrONa (1,5eq.) | 20 | 100/0 |
| 4 | PhONa (1,5eq.) | 0 | - |
| 5 | $CF_3CH_2ONa$ (1,5eq.) | 0 | - |
| 6 | $(Me_3Si)_3N$ (1,5eq.) + MeONa (1,5eq.) | 80 | 100/0 |
| 7 | + EtONa (1,5eq.) | 96 | 100/0 |
| 8 | + iPrONa (1.5eq.) | 81 | 100/0 |
| 9 | + tBuONa (1,5eq.) | 87 | 100/0 |

(suite)

| Essai | BASE | Rdt (%) | OH/OSiMe3 |
|---|---|---|---|
| 10 | + tBuOK (0.2eq.) | 23 | 100/0 |
| 11 | + $CF_3CH_2ONa$ (1,5eq.) | 25 | 100/0 |
| 12 | + PhONa 0,5eq.) | 0 | - |
| 13 | + MeONa (0,2eq.) | 39 | n.d. |
| 14 | + MeONa (0,2eq. à -78°C) | 30 | n.d. |

**Procédure utilisant RONa comme base :**

Essais n° 1 à 5

**Procédure B**

Plage de températures pour la trifluorométhylation : - 20°C à + 30°C.

Concentration du substrat : 1 mmol dans 3ml.

Dans un ballon monocol de 5 ml, maintenu sous azote, on charge l'alcool ROH (1,5 mmol) et l'hydrure de sodium (50 %) (72 mg, 1,5 mmol) dans 2ml de DMF anhydre. Après 40 minutes à 60°C, le mélange réactionnel est refroidi à -10°C. Une solution de benzophénone (182 mg, 1 mmol) dans 1 ml de DMF anhydre est introduire à -10°C. Le fluoroforme (400 mg, 5,7 mmol) est ensuite additionné bulle à bulle.

Le mélange réactionnel est agité à -10°C pendant 1 heure. Après remontée à température ambiante, on en effectue un dosage par RMN$^{19}$F en présence d'un étalon interne ($PhOCF_3$).

**Procédure utilisant N $(SiMe_3)_3$ / RONa comme système basique :**

Essais n° 6 à 12

**Procédure B**

Plage de températures pour la trifluorométhylation : -20°C à + 30°C.

Concentration du substrat : 1 mmol dans 3 ml.

Dans un ballon monocol de 5 ml, maintenu sus azote, on charge l'alcool ROH (1,5 mmol), l'hydrure de sodium (50 %) (72 mg, 1,5 mmol) dans 1 ml de DMF anhydre. Après 40 minutes à 60°C le mélange réactionnel est refroidi à -10°C. Une solution de tris-(triméthylsily)amine (350 mg, 1,5 mmol) dans 1 ml de THF anhydre et une solution de benzophénone (182 mg, 1 mmol) dans 1 ml de DMF anhydre sont introduites successivement à -10°C. Le fluoroforme (400 mg, 5,7 mmol) est ensuite additionné bulle à bulle.

Le mélange réactionnel est agité à -10°C pendant 1 heure. Après remontée à température ambiante, on en effectue un dosage par RMN$^{19}$F en présence d'un étalon interne ($PhOCF_3$).

**Procédure utilisant N $(SiMe_3)_3$ /B $M^+$ (acétate et carbonates) comme système basique :**

Essais n° 13 à 15

**Procédure A**

Plage de température pour la trifluorométhylation : - 20 °C à + 30°C.

Concentration du substrat : 1 mmol dans 3 ml.

Dans un ballon monocol de 5 ml, maintenu sous azote, on charge l'agent désilylant B-M+ (1,5 mmol) et la benzophénone (182 mg, 1 mmol) dans 2 ml de DMF anhydre. Le mélange réactionnel est refroidi à - 10°C. Une solution de tris-(triméthylsilyl)amine (350 mg, 1,5 mmol) dans 1 ml de THF est introduite à - 10°C. Le fluoroforme (400 mg, 5,7 mmol) est ensuite additionné bulle à bulle.

Le mélange réactionnel est agité à -10°C pendant 1 heure. Après remontée à température ambiante, on en effectue un dosage par RMN$^{19}$F en présence d'un étalon interne ($PhOCF_3$).

**Revendications**

1. Composé de formule :

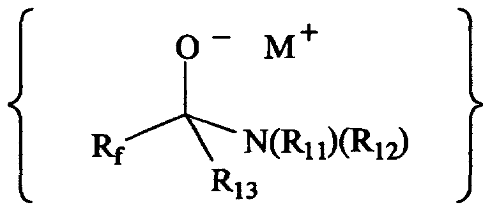

(formule IV)

où $M^+$ représente un cation monovalent avantageusement choisi parmi les alcalins, les phosphoniums et les ammoniums quaternaires ;
où $R_{11}$, $R_{12}$ et $R_{13}$ représentent des chaînes hydrocarbonées, ces chaînes pouvant être reliées entre elles pour former un (voire plusieurs) cycle(s); $R_{13}$ pouvant également prendre la valeur hydrogène ;
où $R_f$ est un radical de formule :

$$GEA\text{-}(CX_2)_P\text{-} \qquad (II)$$

où les X, semblables ou différents, représentent un fluor ou un radical de formule CnF2n + 1, avec n entier au plus égal à 5, de préférence à 2, voire un chlore ;
où p représente un entier au moins égal à 1 et au plus égal à 2 ;
où GEA représente un groupe électro-attracteur dont la constante de Hammett $\sigma_P$ est au moins égale à 0, avec la condition que $R_f$, $R_{13}$, $R_{11}$ et $R_{12}$ ne peuvent pas être simultanément, respectivement, trifluorométhyle, hydrogène, éthyle et méthyle ;

le nombre de carbones global dudit composé étant au plus égal à 50.

2. Composé selon la revendication 1, **caractérisé par le fait que** $R_{13}$ est choisi parmi les radicaux alcoyle, l'hydrogène et les radicaux aryle, avantageusement, dont la valeur absolue de la constante $\sigma_P$ de Hammett est au plus égale à 0,2.

3. Composé selon les revendications 1 et 2, **caractérisé par le fait que** les radicaux $R_{11}$ et $R_{12}$ sont choisis parmi les alcoyles et les aryles.

4. Composé selon les revendications 1 à 3, **caractérisé par le fait que** $R_{11}$ et $R_{12}$ sont alcoyle et que leur nombre de carbones est au plus égal à 6.

5. Réactif utile pour la perfluoroalcoylation, **caractérisé par le fait qu'**il comporte au moins un des composés des revendications 1 à 4.

6. Réactif selon la revendication 5, **caractérisé par le fait que** ledit composé de formule (IV) est présent à une concentration au moins égale à 1 mmol/l, avantageusement à 5 mmol/l, de préférence à 10 mmol/l.

7. Réactif utile pour l'obtention de dérivés perfluoroalcoylés et utiles pour préparer le réactif selon les revendications 5 et 6, **caractérisé par le fait qu'**il comporte pour addition successive ou simultanée :

- une base silicophile ;
- un matériau de formule $R_f$-H ; et
- un dérivé azoté trivalent persilylé.

8. Réactif selon la revendication 7, **caractérisé par le fait que** ledit réactif comporte en outre un solvant (ou mélange de solvants) polaire, et dont la première facidité soit telle que son pKa soit au moins égal à 20, avantageusement à 25, de préférence compris entre 25 et 35.

**9.** Réactif selon les revendications 7 et 8, **caractérisé par le fait que** ledit réactif comporte en outre au moins un composé porteur, d'au moins une double liaison de type C = Y, où Y représente un azote avantageusement substitué ou un atome de chalcogène.

**10.** Réactif selon les revendications 7 à 9, **caractérisé par le fait que** le (ou les) cation(s) associé(s) à ladite base silicophile est choisi(s) parmi les ammoniums quaternaires, les phosphoniums quaternaires et les alcalins.

**11.** Réactif selon les revendications 7 à 10, **caractérisé par le fait que** le (ou les) cation(s) associé(s) à ladite base silicophile est (sont) choisi(s) parmi les alcalins de nombre atomique au moins égal, de préférence supérieur, à celui du sodium.

**12.** Réactif selon les revendications 7 à 11, **caractérisé par le fait que** l'anion de ladite base silicophile est choisi parmi le fluorure et ses complexes, les anions alcoolates et leurs mélanges.

**13.** Réactif selon les revendications 7 à 12, **caractérisé par le fait que** ledit dérivé azoté trivalent persilylé est choisi parmi l'ammoniac et les amines primaires persilylés et leurs mélanges.

**14.** Réactif selon les revendications 5 et 6, **caractérisé par le fait qu'**il comporte en outre un solvant.

**15.** Réactif selon les revendications 8 à 14, **caractérisé par le fait que** ledit solvant est avantageusement choisi parmi les éthers et ceux des amines qui n'ont pas d'hydrogène acide et leurs mélanges.

**16.** Réactif selon les revendications 5 à 15, **caractérisé par le fait que** ledit solvant est avantageusement choisi parmi ceux des amides qui n'ont pas d'hydrogène acide.

**17.** Utilisation des réactifs selon les revendications 1 à 16, pour réaliser une réaction de perfluoroalcoylation.

**18.** Composé répondant à la formule $R_1,R_2,R_f,C\text{-}Y\text{-}R_{20}$, où $R_{20}$ est un radical, avantageusement d'au plus 10 carbones, choisi parmi les alcoyles, les silyles, les acyles, celui correspondant aux combinaisons bisulfitiques ;

où Y représente un azote avantageusement substitué ou un atome de chalcogène ;
où $R_1$ est un radical choisi parmi l'hydrogène, les radicaux hydrocarbonés tels que aryle et alcoyle ;
où $R_2$ est un radical choisi parmi les fonctions amines persubstituées.

**19.** Composé selon la revendication 18, répondant à la formule $R_1,R_2,R_f,C\text{-}Y\text{-}R_{20}$, **caractérisé par le fait que** Y est un atome de chalcogène.

**20.** Procédé de préparation du réactif de perfluoroalcoylation selon les revendications 5 à 16, **caractérisé par le fait qu'**il consiste à faire réagir une base dont l'anion est silicophile, avec un composé de formule $R_f$-H en présence d'un dérivé azoté trivalent persilylé, avantageusement en quantités au moins stoechiométriques par rapport à la base ; où $R_f$ est un radical de formule :

$$GEA\text{-}(CX_2)_P\text{-} \qquad (II)$$

où les X, semblables et différents, représentent un fluor ou un radical de formule $CNF_2N + 1$, avec N entier au plus égal à 5, de préférence en 2, voire un chlore ;
où p représente un entier au moins égal à 1 et au plus égal à 2 ;
où GEA représente un groupe électro-attracteur dont la constante de Hammett $\sigma_P$ est au moins égale à 0, avec la condition que $R_f$, $R_{13}$, $R_{11}$ et $R_{12}$ ne peuvent pas être simultanément, respectivement, trifluorométhyle, hydrogène, éthyle et méthyle ;

le nombre de carbones global dudit composé $R_f$-H étant au plus égal à 50

**21.** Procédé selon la revendication 20, **caractérisé par le fait que** ladite base silicophile est choisie parmi les fluorures et les alcoolates de pKa au moins égal à 6, avantageusement à 8, de préférence à 9.

**22.** Procédé selon les revendications 21 et 22, **caractérisé par le fait que** ladite base silicophile est choisie parmi les fluorures et les alcoolates de formule $R_f(R_5)(R_6)C\text{-}O^-$, où $R_f$ a déjà été défini et/ou $R_5$ et $R_6$ sont choisis parmi l'hydrogène et des restes hydrocarbonés.

**23.** Procédé de préparation d'un réactif de perfluoroalcoylation selon les revendications 5 à 16, **caractérisé par le fait que** l'on soumet un composé de formule $R_f(R_1)(R_2)C\text{-}OZ'$, où Z' est choisi parmi l'hydrogène et $-Si(R')_3$, où les R' semblables ou différents sont des radicaux hydrocarbonés de 1 à environ 10 atomes de carbone, de préférence de 1 à 5, à une base avantageusement silicophile dans le cas où Z' est silyle.

**24.** Procédé selon la revendication 23, **caractérisé par le fait que** Z' est hydrogène et forme un hémiaminal et **par le fait que** ledit hémiaminal est obtenu par action d'une amine secondaire sur l'aldéhyde de formule $R_f$-CHO, avec déshydratation.

**25.** Procédé selon les revendications 23 et 24, **caractérisé par le fait que** Z' est égal à un hydrogène et forme un hémiaminal et **par le fait que** ledit hémiaminal est obtenu par action d'une amine secondaire sur l'aldéhyde de formule $R_f$-CHO, avec déshydratation, et **par le fait que** la déshydratation est réalisée par distillation hétéro(azéotropique) de l'eau.

## Patentansprüche

**1.** Verbindung der Formel:

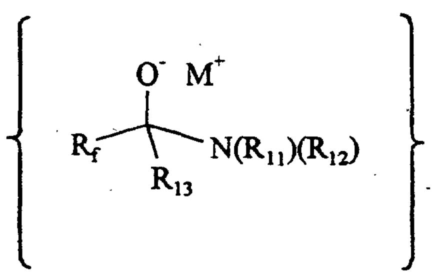

**(Formel IV),**

in der:

- $M^+$ ein einwertiges Kation darstellt, das vorteilhafterweise unter den Alkalikationen, den Phosphoniumkationen und den quartären Ammoniumkationen ausgewählt ist;
- $R_{11}$, $R_{12}$ und $R_{13}$ Kohlenwasserstoffketten darstellen, wobei diese Ketten unter Bildung eines (oder auch mehrerer) Ringe(s) miteinander verbunden sein können und wobei $R_{13}$ auch ein Wasserstoffatom darstellen kann;
- $R_f$ ein Rest der folgenden Formel ist:

$$GEA\text{-}(CX_2)_p\text{-} \qquad (II),$$

in der:

- die Reste X, die gleich oder verschieden sein können, jeweils ein Fluoratom, einen Rest der Formel $C_nF_{2n+1}$, wobei in diesem Rest n eine ganze Zahl kleiner oder gleich 5 und vorzugsweise gleich 2 darstellt, oder ein Chloratom darstellen;
- p eine ganze Zahl größer oder gleich 1 und kleiner oder gleich 2 darstellt;
- GEA eine elektronenziehende Gruppe darstellt, deren Hammett-Konstante $\sigma_P$ größer oder gleich 0 ist,

mit der Maßgabe, dass $R_f$, $R_{13}$, $R_{11}$ und $R_{12}$ nicht gleichzeitig Trifluormethyl, Wasserstoff, Ethyl oder Methyl darstellen können;
wobei die Gesamtzahl der Kohlenstoffatome dieser Verbindung maximal 50 beträgt.

2. Verbindung nach Anspruch 1, **dadurch gekennzeichnet, dass** $R_{13}$ unter den Alkylresten, dem Wasserstoffatom und den Arylresten ausgewählt ist, deren Absolutwert der Hammett-Konstante $\sigma_P$ vorzugsweise kleiner oder gleich 0,2 ist.

3. Verbindung nach den Ansprüchen 1 und 2, **dadurch gekennzeichnet, dass** die Reste $R_{11}$ und $R_{12}$ unter den Alkyl- und Arylresten ausgewählt sind.

4. Verbindung nach den Ansprüchen 1 bis 3, **dadurch gekennzeichnet, dass** $R_{11}$ und $R_{12}$ Alkylreste sind, deren Anzahl an Kohlenstoffatomen maximal 6 beträgt.

5. Reagenz, das zur Perfluoralkylierung verwendet werden kann, **dadurch gekennzeichnet, dass** es mindestens eine der Verbindungen entsprechend den Ansprüchen 1 bis 4 enthält.

6. Reagenz nach Anspruch 5, **dadurch gekennzeichnet, dass** die besagte Verbindung der Formel (IV) in einer Konzentration größer oder gleich 1 mmol/l, vorteilhafterweise größer oder gleich 5 mmol/l und vorzugsweise größer oder gleich 10 mmol/l vorliegt.

7. Reagenz, das zur Darstellung von perfluoralkyliertenVerbindungen verwendet werden kann, aus welchen das Reagenz entsprechend den Ansprüchen 5 und 6 hergestellt werden kann, **dadurch gekennzeichnet, dass** es zur sukzessiven oder gleichzeitigen Addition folgende Komponenten aufweist:

- eine silicophile Base;
- eine Verbindung der Formel $R_f$-H und
- ein persilyliertes dreiwertiges Stickstoffderivat.

8. Reagenz nach Anspruch 7, **dadurch gekennzeichnet, dass** dieses Reagenz zusätzlich ein polares Lösungsmittel (oder Lösungsmittelgemisch) enthält, das sich in erster Linie durch einen $pK_a$-Wert größer oder gleich 20, vorteilhafterweise größer oder gleich 25 und vorzugsweise im Bereich von 25 bis 35 auszeichnet.

9. Reagenz nach den Ansprüchen 7 und 8, **dadurch gekennzeichnet, dass** dieses Reagenz zusätzlich mindestens eine Verbindung enthält, die mindestens eine Doppelbindung des Typs C=Y aufweist , wobei Y ein Stickstoffatom, das vorteilhafterweise substituiert ist, oder ein Chalcogenatom darstellt.

10. Reagenz nach den Ansprüchen 7 bis 9, **dadurch gekennzeichnet, dass** das (beziehungsweise die) mit der besagten silicophilen Base assoziierte(n) Kation(en) unter den quartären Ammoniumkationen, den quartären Phosphoniumkationen und den Alkalikationen ausgewählt ist (sind).

11. Reagenz nach den Ansprüchen 7 bis 10, **dadurch gekennzeichnet, dass** das (beziehungsweise die) mit der besagten silicophilen Base assoziierte(n) Kation(en) unter den Alkalikationen ausgewählt ist (sind), deren Ordnungszahl größer oder gleich und vorzugsweise größer als die Ordnungszahl des Natriums ist.

12. Reagenz nach den Ansprüchen 7 bis 11, **dadurch gekennzeichnet, dass** das Anion der besagten silicophilen Base unter dem Fluorid und seinen Komplexen, den Alkoholatanionen und deren Gemischen ausgewählt ist.

13. Reagenz nach den Ansprüchen 7 bis 12, **dadurch gekennzeichnet, dass** das besagte persilylierte dreiwertige Stickstoffderivat unter persilyliertem Ammoniak, den persilylierten primären Aminen und deren Gemischen ausgewählt ist.

14. Reagenz nach den Ansprüchen 5 und 6, **dadurch gekennzeichnet, dass** es zusätzlich ein Lösungsmittel enthält.

15. Reagenz nach den Ansprüchen 8 bis 14, **dadurch gekennzeichnet, dass** das besagte Lösungsmittel vorteilhafterweise unter den Ethern und den Lösungsmitteln für Amine, die kein saures Wasserstoffatom aufweisen, sowie deren Gemischen ausgewählt ist.

16. Reagenz nach den Ansprüchen 5 bis 15, **dadurch gekennzeichnet, dass** das besagte Lösungsmittel vorteilhafterweise unter den Lösungsmitteln für Amide, die kein saures Wasserstoffatom aufweisen, ausgewählt ist.

17. Verwendung der Reagenzien entsprechend den Ansprüchen 1 bis 16 zur Durchführung einer Perfluoralkylierungsreaktion.

**18.** Verbindung entsprechend der Formel $R_1$,$R_2$,$R_f$,C-Y-$R_{20}$, in der $R_{20}$ ein Rest ist, der vorteilhafterweise maximal 10 Kohlenstoffatome aufweist und der ausgewählt ist unter den Alkylresten, den Silykesten und den Acylresten (dieses entsprechend den Bisulfit-Addukten);
wobei

- Y ein Stickstoffatom, das vorteilhafterweise substituiert ist, oder ein Chalcogenatom darstellt;
- $R_1$ unter dem Wasserstoffatom und den Kohlenwasserstoffresten, wie den Aryl- und Alkylresten, ausgewählter Rest ist und
- $R_2$ ein unter den persubstituierten Aminogruppen ausgewählter Rest ist.

**19.** Verbindung nach Anspruch 18 entsprechend der Formel $R_1$,$R_2$,$R_f$,C-Y-$R_{20}$, **dadurch gekennzeichnet, dass** Y ein Chalcogenatom ist.

**20.** Verfahren zur Herstellung des Perfluoralkylierungsreagenzes entsprechend den Ansprüchen 5 bis 16, **dadurch gekennzeichnet, dass** dieses Verfahren darin besteht, eine Base, die ein silicophiles Anion aufweist, in Gegenwart eines persilylierten dreiwertigen Stickstoffderivats mit einer Verbindung der Formel $R_f$-H umzusetzen, wobei die Verbindung der Formel $R_f$-H und das persilylierte dreiwertige Stickstoffderivat vorteilhafterweise in mindestens stöchiometrischer Menge, bezogen auf die Base, eingesetzt werden und wobei $R_f$ ein Rest der folgenden Formel ist:

$$GEA\text{-}(CX_2)_p\text{-} \qquad (II),$$

in der:

- die Reste X, die gleich oder verschieden sein können, jeweils ein Fluoratom, einen Rest der Formel $C_nF_{2n+1}$, wobei in diesem Rest n eine ganze Zahl kleiner oder gleich 5 und vorzugsweise gleich 2 darstellt, oder ein Chloratom darstellen;
- p eine ganze Zahl größer oder gleich 1 und kleiner oder gleich 2 darstellt;
- GEA eine elektronenziehende Gruppe darstellt, deren Hammett-Konstante $\sigma_P$ größer oder gleich 0 ist,

mit der Maßgabe, dass $R_f$, $R_{13}$, $R_{11}$ und $R_{12}$ nicht gleichzeitig Trifluormethyl, Wasserstoff, Ethyl oder Methyl darstellen können.

**21.** Verfahren nach Anspruch 20, **dadurch gekennzeichnet, dass** die besagte silicophile Base unter den Fluoriden und den Alkoholaten ausgewählt ist, die einen $pK_a$-Wert größer oder gleich 6, vorteilhafterweise größer oder gleich 8 und vorzugsweise größer oder gleich 9 aufweisen.

**22.** Verfahren nach den Ansprüchen 21 und 22, **dadurch gekennzeichnet, dass** die besagte silicophile Base unter den Fluoriden und den Alkoholaten der Formel $R_f(R_5)(R_6)C\text{-}O^-$ ausgewählt ist, wobei $R_f$ bereits definiert wurde und/oder $R_5$ und $R_6$ unter dem Wasserstoffatom und den Kohlenwasserstoffresten ausgewählt sind.

**23.** Verfahren zur Herstellung eines Perfluoralkylierungsreagenzes nach den Ansprüchen 5 bis 16, **dadurch gekennzeichnet, dass** eine Verbindung der Formel $R_f(R_1)(R_2)C\text{-}OZ'$, in der Z' unter dem Wassserstoffatom und -Si(R')$_3$ ausgewählt ist, wobei die Reste R', die gleich oder verschieden sein können, Kohlenwasserstoffreste mit 1 bis ungefähr 10 und vorzugsweise 1 bis 5 Kohlenstoffatomen sind, mit einer Base umgesetzt wird, die in den Fällen, in denen Z' ein Silylrest ist, vorzugsweise silicophil ist.

**24.** Verfahren nach Anspruch 23, **dadurch gekennzeichnet, dass** Z' ein Wasserstoffatom ist, das ein Halbaminal bildet, und dass dieses Halbaminal durch Umsetzung eines sekundären Amins mit dem Aldehyd der Formel $R_f$-CHO und Dehydratisierung gebildet wird.

**25.** Verfahren nach den Ansprüchen 23 und 24, **dadurch gekennzeichnet, dass** Z' ein Wasserstoffatom ist, das ein Halbaminal bildet, und dass dieses Halbaminal durch Umsetzung eines sekundären Amins mit dem Aldehyd der Formel $R_f$-CHO und Dehydratisierung gebildet wird, wobei diese Dehydratisierung durch heteroazeotropes Abdestillieren des Wassers erfolgt.

## Claims

1. Compound of formula:

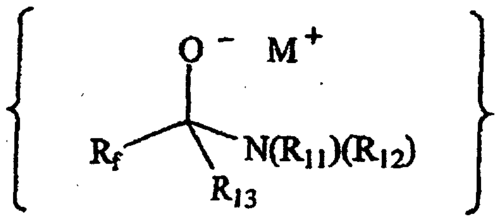

(formula IV)

where $M^+$ represents a monovalent cation advantageously chosen from alkali metals, quaternary phosphoniums and quaternary ammoniums;
where $R_{11}$, $R_{12}$ and $R_{13}$ represent hydrocarbonaceous chains, it being possible for these chains to be connected to one another to form a (or indeed several) ring(s); it also being possible for $R_{13}$ to be hydrogen;
where $R_f$ is a radical of formula:

$$EWG\text{-}(CX_2)_P\text{-} \quad (II)$$

where the X groups, which are alike or different, represent a fluorine or a radical of formula $C_nF_{2n+1}$, with n an integer at most equal to 5, preferably to 2, indeed even a chlorine;
where p represents an integer at least equal to 1 and at most equal to 2;
where EWG represents an electron-withdrawing group, the $\sigma_P$ Hammett constant of which is at least equal to 0, with the condition that $R_f$, $R_{13}$, $R_{11}$ and $R_{12}$ cannot simultaneously be, respectively, trifluoromethyl, hydrogen, ethyl and methyl;
the total number of carbons of the said compound being at most equal to 50.

2. Compound according to Claim 1, **characterized in that** $R_{13}$ is chosen from alkyl radicals, hydrogen and aryl radicals, advantageously, the absolute value of the $\sigma_P$ Hammett constant of which is at most equal to 0.2.

3. Compound according to Claims 1 and 2, **characterized in that** the $R_{11}$ and $R_{12}$ radicals are chosen from alkyls and aryls.

4. Compound according to Claims 1 to 3, **characterized in that** $R_{11}$ and $R_{12}$ are alkyl and **in that** their number of carbons is at most equal to 6.

5. Reagent of use for perfluoroalkylation, **characterized in that** it comprises at least one of the compounds of Claims 1 to 4.

6. Reagent according to Claim 5, **characterized in that** the said compound of formula (IV) is present at a concentration at least equal to 1 mmol/l, advantageously to 5 mmol/l, preferably to 10 mmol/l.

7. Reagent of use for the preparation of perfluoroalkylated derivatives which are of use for preparing the reagent according to Claims 5 and 6, **characterized in that** it comprises, for successive or simultaneous addition:

- a silicophilic base;
- a material of formula $R_f$-H; and
- a persilylated trivalent nitrogenous derivative.

**8.** Reagent according to Claim 7, **characterized in that** the said reagent additionally comprises a polar solvent (or mixture of polar solvents), the first acidity of which is such that its pKa is at least equal to 20, advantageously to 25, preferably between 25 and 35.

**9.** Reagent according to Claims 7 and 8, **characterized in that** the said reagent additionally comprises at least one compound carrying at least one double bond of C=Y type, where Y represents an advantageously substituted nitrogen or a chalcogen atom.

**10.** Reagent according to Claims 7 to 9, **characterized in that** the cation(s) associated with the said silicophilic base is (are) chosen from quaternary ammoniums, quaternary phosphoniums and alkali metals.

**11.** Reagent according to Claims 7 to 10, **characterized in that** the cation(s) associated with the said silicophilic base is (are) chosen from alkali metals with an atomic number at least equal to, preferably greater than, that of sodium.

**12.** Reagent according to Claims 7 to 11, **characterized in that** the anion of the said silicophilic base is chosen from fluoride and its complexes, alkoxide anions and their mixtures.

**13.** Reagent according to Claims 7 to 12, **characterized in that** the said persilylated trivalent nitrogenous derivative is chosen from persilylated ammonia and primary amines and their mixtures.

**14.** Reagent according to Claims 5 and 6, **characterized in that** it additionally comprises a solvent.

**15.** Reagent according to Claims 8 to 14, **characterized in that** the said solvent is advantageously chosen from ethers and those of the amines which do not have acidic hydrogen and their mixtures.

**16.** Reagent according to Claims 5 to 15, **characterized in that** the said solvent is advantageously chosen from those of the amides which do not have acidic hydrogen.

**17.** Use of the reagents according to Claims 1 to 16 for carrying out a perfluoroalkylation reaction.

**18.** Compound corresponding to the formula $R_1R_2R_fC\text{-}Y\text{-}R_{20}$,
where $R_{20}$ is a radical, advantageously of at most 10 carbons, chosen from alkyls, silyls, acyls, that corresponding to the bisulphite combinations;
where Y represents an advantageously substituted nitrogen or a chalcogen atom;
where $R_1$ is a radical chosen from hydrogen or hydrocarbonaceous radicals, such as aryl and alkyl;
where $R_2$ is a radical chosen from persubstituted amine functional groups.

**19.** Compound according to Claim 18 corresponding to the formula $R_1R_2R_fC\text{-}Y\text{-}R_{20}$, **characterized in that** Y is a chalcogen atom.

**20.** Process for the preparation of the perfluoroalkylation reagent according to Claims 5 to 16, **characterized in that** it consists in reacting a base, the anion of which is silicophilic, with a compound of formula $R_f\text{-}H$ in the presence of a persilylated trivalent nitrogenous derivative, advantageously in amounts which are at least stoichiometric with respect to the base; where $R_f$ is a radical of formula:

$$EWG\text{-}(CX_2)_P\text{-} \qquad \text{(II)}$$

where the X groups, which are alike or different, represent a fluorine or a radical of formula $C_nF_{2n+1}$, with n an integer at most equal to 5, preferably to 2, indeed even a chlorine;
where p represents an integer at least equal to 1 and at most equal to 2;
where EWG represents an electron-withdrawing group, the $\sigma_P$ Hammett constant of which is at least equal to 0, with the condition that $R_f$, $R_{13}$, $R_{11}$ and $R_{12}$ cannot simultaneously be, respectively, trifluoromethyl, hydrogen, ethyl and methyl.

**21.** Process according to Claim 20, **characterized in that** the said silicophilic base is chosen from fluorides and alkoxides with a pKa at least equal to 6, advantageously to 8, preferably to 9.

**22.** Process according to Claims 21 and 22,
**characterized in that** the said silicophilic base is chosen from fluorides and alkoxides of formula $R_f(R_5)(R_6)C\text{-}O^-$, where $R_f$ has already been defined and/or $R_5$ and $R_6$ are chosen from hydrogen and hydrocarbonaceous residues.

**23.** Process for the preparation of a perfluoroalkylation reagent according to Claims 5 to 16, **characterized in that** a radical of formula $R_f(R_1)(R_2)C\text{-}OZ'$, where Z' is chosen from hydrogen and $-Si(R')_3$, where the R' groups, which are alike or different, are hydrocarbonaceous radicals comprising 1 to approximately 10 carbon atoms, preferably comprising 1 to 5 carbon atoms, is subjected to a base which is advantageously silicophilic in the case where Z' is silyl.

**24.** Process according to Claim 23, **characterized in that** Z' is hydrogen and forms a hemiaminal and **in that** the said hemiaminal is obtained by reaction of a secondary amine with the aldehyde of formula $R_f\text{-}CHO$, with dehydration.

**25.** Process according to Claims 23 and 24, **characterized in that** Z' is a hydrogen and forms a hemiaminal and **in that** the said hemiaminal is obtained by reaction of a secondary amine with the aldehyde of formula $R_f\text{-}CHO$, with dehydration, and **in that** the dehydration is carried out by hetero(azeotropic) distillation of the water.